(19) Europäisches Patentamt
European Patent Office
Office européen des brevets

(11) Veröffentlichungsnummer: **0 231 510**
**B1**

(12) **EUROPÄISCHE PATENTSCHRIFT**

(45) Veröffentlichungstag der Patentschrift: **26.09.90**

(21) Anmeldenummer: **86117971.1**

(22) Anmeldetag: **23.12.86**

(51) Int. Cl.[5]: **C 07 D 231/18,**
C 07 D 401/04, A 01 N 43/56,
A 01 N 43/40

# (54) 1-Arylpyrazole.

(30) Priorität: **08.01.86 DE 3600287**

(43) Veröffentlichungstag der Anmeldung:
**12.08.87 Patentblatt 87/33**

(45) Bekanntmachung des Hinweises auf die Patenterteilung:
**26.09.90 Patentblatt 90/39**

(84) Benannte Vertragsstaaten:
**AT BE CH DE ES FR GB GR IT LI NL SE**

(56) Entgegenhaltungen:
**EP-A- 138 527**
**EP-A- 200 872**
**EP-A- 201 852**

**CHEMICAL ABSTRACTS, Band 85, Nr. 21, 22. November 1976, Seite 551, Zusammenfassung Nr. 160084p, Columbus, Ohio, US; & JP-A-76 43 758**

**CHEMICAL ABSTRACTS, Band 85, Nr. 1, 5. Juli 1976, Seite 454, Zusammenfassung Nr. 5620s, Columbus, Ohio, US; & JP-A-75 130 761**

(73) Patentinhaber: **BAYER AG**
**D-5090 Leverkusen 1 Bayerwerk (DE)**

(72) Erfinder: **Jensen-Korte, Uta, Dr.**
**Geibelstrasse 9**
**D-4000 Düsseldorf (DE)**
Erfinder: **Schallner, Otto, Dr.**
**Noldeweg 22**
**D-4019 Monheim (DE)**
Erfinder: **Stetter, Jörg, Dr.**
**Gellertweg 4**
**D-5600 Wuppertal 1 (DE)**
Erfinder: **Wroblowsky, Heinz-Jürgen, Dr.**
**Gladbacher Strasse 34**
**D-4018 Langenfeld (DE)**
Erfinder: **Stendel, Wilhelm, Dr.**
**In den Birken 55**
**D-5600 Wuppertal 1 (DE)**
Erfinder: **Becker, Benedikt, Dr.**
**Metzkausener Strasse 14**
**D-4020 Mettmann (DE)**
Erfinder: **Homeyer, Bernhard, Dr.**
**Obere Strasse 28**
**D-5090 Leverkusen 3 (DE)**
Erfinder: **Behrenz, Wolfgang, Dr.**
**Untergruendemich 14**
**D-5063 Overath (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 231 510 B1**

Courier Press, Leamington Spa, England.

**Beschreibung**

Die Erfindung betrifft neue 1-Arylpyrazole, ein Verfahren zu ihrer Herstellung und ihre Verwendung als Schädlingsbekämpfungsmittel, insbesondere als Insektizide.

Aus den Publikationen JP—A—5143758 ($D_1$), JP—A—50130761 ($D_2$) und EP—A—138527 ($D_3$) sind ähnlich strukturierte Phenylpyrazole bekannt. Diese zeigen jedoch im Gegensatz zu den insektizid wirksamen erfindungsgemäßen Verbindungen entweder analgetische und antipyretische ($D_2$) oder herbizide Eingenschaften ($D_3$).

Weiterhin sind ähnlich strukturierte Phenylpyrazole, welche in 1-Stellung den 4-Nitrophenyl- oder den 2,4-Dinitrophenyl-Rest enthalten aus J. Fluorine Chem. *1980*, 16 (2), 129—136, ohne Wirkungsangabe bekannt.

Aus der Publikation DE—A—2 839 270 ist bereits bekannt, daß bestimmte Pyrazol-Derivate, wie beispielsweise das 1-Cyclohexyl-5-[N,N-(dimethyl)carbamoyloxy]-3-methylthiomethyl-pyrazol, das 1-Cyclohexyl-5-[N,N-(dimethyl)-carbamoyloxy]-3-methylsulfinylmethylpyrazol oder das 1-Cyclohexyl-5-[N,N-(dimethyl)-carbamoyloxy]-3-methylsulfonylmethyl-pyrazol insektizide Wirkung besitzen.

Die Wirkungshöhe bzw. Wirkungsdauer dieser Verbindungen sind jedoch, insbesondere bei bestimmten Insekten oder bei neidrigen Anwendungskonzentrationen nicht immer in allen Anwendungsbereichen völlig zufriedenstellend. Aufgabe der Erfindung war es, neue stark insektizid wirksame Stoffe bereitzustellen.

Es wurden nun neue 1-Aryle-pyrazole der allgemeinen Formel (I),

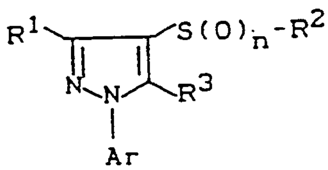

(I)

gefunden,

in welcher

$R^1$ für Wasserstoff, oder für jeweils geradkettiges oder verzweigtes Alkyl oder Halogenalkyl mit 1 bis 4 Kohlenstoffatomen und gegebenenfalls 1 bis 9 gleichen oder verschiedenen Halogenatomen steht,

$R^2$ für jeweils geradkettiges oder verzweigtes Alkyl, Alkenyl, oder Alkinyl mit jeweils bis zu 8 Kohlenstoffatomen, für Cycloalkyl mit 3 bis 7 Kohlenstoffatomen, für jeweils geradkettiges oder verzweigtes Halogenalkyl oder Halogenalkenyl mit jeweils bis zu 8 Kohlenstoffatomen und bis zu 17 gleichen oder verschiedenen Halogenatomen, für jeweils geradkettiges oder verzweigtes Alkoxyalkyl, Alkylthioalkyl, Alkylsulfinylalkyl oder Alkylsulfonylalkyl mit jeweils 1 bis 6 Kohlenstoffatomen in den einzelnen Alkylteilen oder für jeweils im Phenylteil gegebenenfalls einfach oder mehrfach, gleich oder verschieden substituiertes Phenylalkyl oder Phenyl mit gegebenenfalls 1 bis 4 Kohlenstoffatomen im geradkettigen oder verzweigten Alkylteil steht, wobei als Substituenten im Phenylteil infrage kommen: Halogen, Cyano, Nitro, jeweils geradkettiges oder verzweigtes Alkyl, Alkoxy, Alkylthio, Alkylsulfinyl, Alkylsulfonyl, Halogenalkyl, Halogenalkoxy, Halogenalkylthio, Halogenalkylsulfinyl oder Halogenalkylsulfonyl mit jeweils 1 bis 4 Kohlenstoffatomen in den einzelnen Alkylteilen und gegebenenfalls 1 bis 9 gleichen oder verschiedenen Halogenatomen,

$R^3$ für jeweils geradkettiges oder verzweigtes Alkyl oder Halogenalkyl mit jeweils 1 bis 6 Kohlenstoffatomen und gegebenenfalls 1 bis 12 gleichen oder verschiedenen Halogenatomen, für Cycloalkyl mit 3 bis 7 Kohlenstoffatomen oder für gegebenenfalls einfach oder mehrfach, gleich oder verschieden substituiertes Phenyl steht, wobei als Phenylsubstituenten die bei $R^2$ genannten infrage kommen,

Ar für einfach oder mehrfach, gleich oder verschieden substituiertes Phenyl mit Ausnahme des 4-Nitrophenyl-sowie des 2,4-Dinitrophenylrestes steht, wobei als Substituenten jeweils in Frage kommen: Cyano, Nitro, Halogen, jeweils geradkettiges oder verzweigtes Alkyl, alkoxy oder Alkoxycarbonyl mit jeweils 1 bis 4 Kohlenstoffatomen, außerdem jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen oder ein Rest $—S(O)_p-R^5$, wobei

$R^5$ für Amino sowie für jeweils geradkettiges oder verzweigtes Alkyl, Alkylamino, Dialkylamino oder Halogenalkyl mit jeweils 1 bis 4 Kohlenstoffatomen in den einzelnen Alkylteilen und im Fall des Halogenalkyl mit 1 bis 9 gleichen oder verschiedenen Halogenatomen steht,

p für eine Zahl 0, 1 oder 2 steht und

n für eine Zahl 0, 1 oder 2 steht.

Weiterhin wurde gefunden, daß man die neuen 1-Aryl-pyrazole der allgemeinen Formel (I)

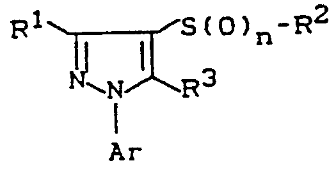

(I)

in welcher $R^1$, $R^2$, $R^3$ Ar und n die oben angegebene Bedeutung besitzen, nach einem der im folgenden beschriebenen Herstellungsverfahren erhält.

Man erhält 1-Aryl-pyrazole der Formel (Ia),

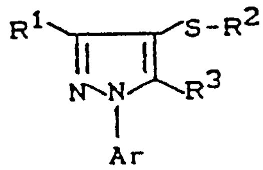

(Ia)

in welcher

$R^1$, $R^2$, $R^3$ und Ar die oben angegebene Bedeutung haben, wenn man

a) Arylhydrazine der Formel (II),

$$Ar{-}NH{-}NH_2 \qquad (II)$$

in welcher

Ar die oben angegebene Bedeutung hat, oder deren Hydrosalze mit 1,3-Diketonen der Formel (IIIa)

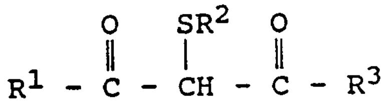

(IIIa)

in welcher

$R^1$, $R^2$ und $R^3$ die oben angegebene Bedeutung haben, oder alternativ mit αβ-ungesättigten Ketonen der Formel (IIIb),

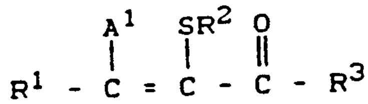

(IIIb)

in welcher

$R^1$, $R^2$ und $R^3$ die oben angegebene Bedeutung haben und

$A^1$ für eine Abgangsgruppe wie beispielsweise Alkoxy oder Dialkylamino steht,

gegebenenfalls in Gegenwart eines Verdünnungsmittels und gegebenenfalls in Gegenwart eines Katalysators umsetzt.

Man erhält 1-Arylpyrazole der Formel (Ib),

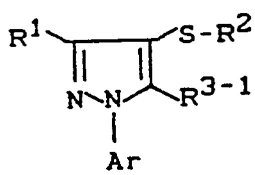

(Ib)

in welcher

$R^1$, $R^2$ und Ar die oben angegebene Bedeutung haben und

$R^{3-1}$ für gegebenenfalls substituiertes Aryl steht,

alternativ auch, wenn man

b) 5-Amino-1-aryl-pyrazole der Formel (IV),

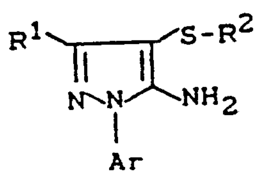

(IV)

mit Aromaten der Formel (V),

$$R^{3-1}{-}H \qquad (V)$$

in welcher

$R^{3-1}$ die oben angegebene Bedeutung hat,

in Gegenwart eines Alkylnitrits der Formel (VI),

$$R^4—O—N=O \qquad (VI)$$

in welcher

$R^4$ für Alkyl steht

und gegebenenfalls in Gegenwart eines Verdünnungsmittels umsetzt.

Man erhält 4-Sulfinyl- und 4-Sulfonyl-1-aryl-pyrazole der Formel (Ic),

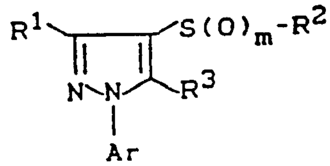

(Ic)

in welcher

$R^1$, $R^2$, $R^3$ und Ar die oben angegebene Bedeutung haben und

m für ene Zahl 1 oder 2 steht,

wenn man

c) die nach Verfahren (a) oder (b) erhältlichen 1-Arylpyrazole der Formel (Ia),

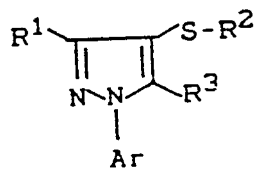

(Ia)

in welcher

$R^1$, $R^2$, $R^3$ und Ar die oben angegebene Bedeutung haben,

mit Oxidationsmitteln gegebenenfalls in Gegenwart eines Verdünnungsmittels und gegebenenfalls in Gegenwart eines Katalysators in üblicher Art und Weise oxidiert.

Schließlich wurde gefunden, daß die neuen 1-Aryl-pyrazole der allgemeinen Formel (I) pestizide und insbesondere insektizide Eigenschaften besitzen.

Überraschenderweise zeigen die erfindungsgemäßen 1-Arylpyrazole der allgemeinen Formel (I) eine erheblich bessere insektizide Wirksamkeit, als die aus dem Stand der Technik DE—A—2 839 270 bekannten Pyrazol-Derivate, wie beispielsweise das 1-Cyclohexyl-5-[N,N-(dimethyl)-carbamoyloxy]-3-methylthiomethyl-pyrazol oder das 1-Cyclohexyl-5-[N,N-(dimethyl)carbamoyloxy]-3-methylsulfonyl-methyl-pyrazol, welche chemisch und wirkungsmäßig naheliegende Verbindungen sind.

Die erfindungsgemäßen 1-Aryl-pyrazole sind durch die Formel (I) allgemein definiert. Bevorzugt sind Verbindungen der Formel (I), bei welchen

$R^1$ für Wasserstoff, Methyl, Ethyl, n- oder i-Propyl oder Trifluormethyl steht,

$R^2$ für Methyl, Ethyl, n- oder i-Propyl, n-, i-, s- oder t-Butyl, N- oder i-Pentyl, n- oder i-Hexyl, Allyl, n- oder i-Butenyl, N- oder i-Pentenyl, Propargyl, n- oder i-Butinyl, Cyclopropyl, Cyclobutyl, Cyclopentyl, Cyclohexyl, Chlormethyl, Difluormethyl, Difluorchlormethyl, Fluorichlormethyl, Trifluormethyl, Pentafluorethyl, Pentachlorethyl, Fluortetrachlorethyl, Difluortrichlorethyl, Trifluordichlorethyl, Tetrafluorchlorethyl, Heptafluorpropyl, Chlorethyl, Bromethyl, Chlorpropyl, Brompropyl, Dichlormethyl, Chlorfluormethyl, Trichlormethyl, Dichlormethyl, Trifluorethyl, Trifluorchlorethyl, Tetrafluorethyl, Difluorchlorethyl, Fluordibrommethyl, Difluorbrommethyl, Fluorchlorbrommethyl, Chloralkyl, Fluorallyl, Chlorbutenyl, Fluorbutenyl, Dichlorallyl, Fluorchlorallyl, Difluorallyl, Bromallyl, für Methoxmethyl, Methoxyethyl, Methoxypropyl, Ethoxymethyl, Ethoxyethyl, Methylthiomethyl, Methylsulfinylmethyl, Methylsulfonylethyl, Methylthioethyl, Methylthiopropyl, Methylsulfinylethyl, Methylsulfonylmethyl oder für jeweils gegebenenfalls einfach bis dreifach, gleich oder verschieden substituiertes Phenyl, Benzyl oder Phenylethyl steht, wobei als Phenylsubstituenten in Frage kommen: Fluor, Chlor, Brom, Iod, Cyano, Nitro, Methyl, Ethyl, Methoxy, Methylthio, Trifluormethyl, Methylsulfinyl, Methylsulfonyl, Trifluormethoxy, Trifluormethylthio, Trifluormethylsulfinyl oder Trifluormethylsulfonyl,

$R^3$ für Methyl, Ethyl, n- oder i-Propyl, n-, i-, s- oder t-Butyl, Cyclopropyl, Cyclobutyl, Cyclopentyl, cyclohexyl, für Chlormethyl, Dichlormethyl, Trichlormethyl, Brommethyl, Dibrommethyl, Fluormethyl, Difluormethyl, Trifluormethyl, Fluordichlormethyl, Difluorchlormethyl oder für gegebenenfalls ein- bis dreifach, gleich oder verschieden substituierts Phenyl steht, wobei als Phenylsubstituenten die bei $R^2$ genannten infrage kommen,

Ar für eine- bis fünffach, gleich oder verschieden substituiertes Phenyl mit Ausnahme des 4-Nitrophenyl- sowie des 2,4-Dinitrophenylrestes oder für jeweils gegebenenfalls ein- bis vierfach gleich oder

verschieden substituiertes 2-Pyridyl oder 4-Pyridyl steht, wobei als Phenyl- bzw. Pyridylsubstituenten jeweils in Frage kommen: Cyano, Nitro, Fluor, Chlor, Brom, Iod, Methyl, Ethyl, n- und i-Propyl, n-, i-, s- und t-Butyl, Methoxy, Ethoxy, Methoxycarbonyl, Ethoxycarbonyl, Trifluormethyl, Trichlormethyl, Dichlorfluormethyl, Difluorchlormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder ein Rest $—S(O)_p—R^5$,

wobei

$R^5$ für Amino, Methylamino, Ethylamino, Dimethylamino, Diethylamino, Fluordichlormethyl, Difluorchlormethyl, Tetrafluorethyl, Trifluorchlorethyl, Trifluormethyl, Methyl oder Ethyl steht,

p für eine Zahl 0, 1 oder 2 steht und

n für eine Zahl 0, 1 oder 2 steht.

Im einzelnen seien außer den bei den Herstellungsbeispielen genannten Verbindungen die folgenden 1-Arylpyrazole der allgemeinen formel (I) genannt:

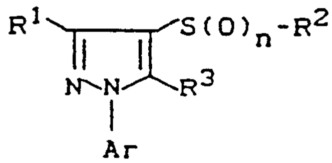

(I)

| $R^1$ | $R^2$ | $R^3$ | n | Ar |
|---|---|---|---|---|
| H | $CH_3$ | $CH_3$ | 0 | 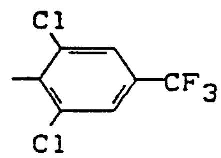 |
| H | $CH_3$ | $CH_3$ | 1 | 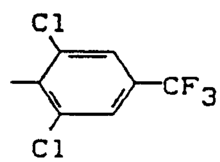 |
| H | $CH_3$ | $CH_3$ | 2 | 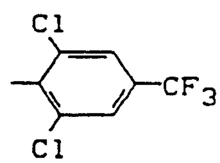 |
| $CH_3$ | $CH_3$ | $CH_3$ | 0. | 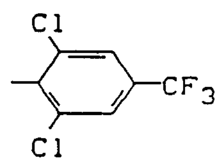 |
| $CH_3$ | $CH_3$ | $CH_3$ | 1 | 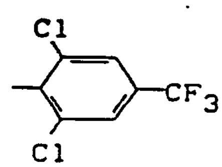 |

| $R^1$ | $R^2$ | $R^3$ | n | Ar |
|---|---|---|---|---|
| $CH_3$ | $CH_3$ | $CH_3$ | 2 | 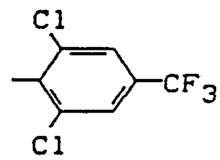 |
| H | $C_2H_5$ | $CH_3$ | 0 | 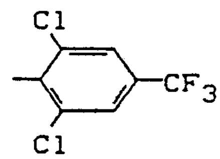 |
| H | $C_2H_5$ | $CH_3$ | 1 | 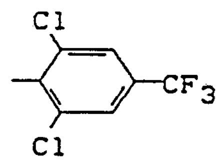 |
| H | $C_2H_5$ | $CH_3$ | 2 | 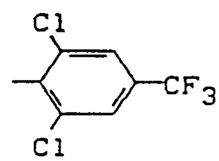 |
| $CH_3$ | $C_2H_5$ | $CH_3$ | 0 | 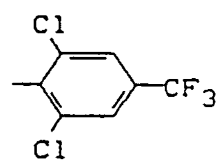 |
| $CH_3$ | $C_2H_5$ | $CH_3$ | 1 | 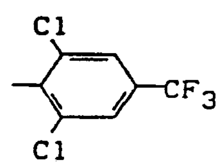 |
| $CH_3$ | $C_2H_5$ | $CH_3$ | 2 | 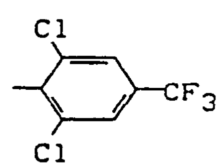 |
| H | $i-C_3H_7$ | $CH_3$ | 0 | 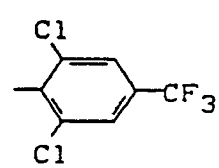 |

| $R^1$ | $R^2$ | $R^3$ | n | Ar |
|---|---|---|---|---|
| H | $i\text{-}C_3H_7$ | $CH_3$ | 1 | 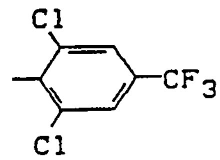 |
| H | $i\text{-}C_3H_7$ | $CH_3$ | 2 | 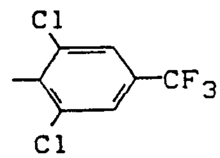 |
| $CH_3$ | $i\text{-}C_3H_7$ | $CH_3$ | 0 | 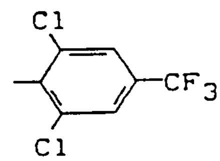 |
| $CH_3$ | $i\text{-}C_3H_7$ | $CH_3$ | 1 | 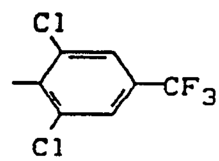 |
| $CH_3$ | $i\text{-}C_3H_7$ | $CH_3$ | 2 | 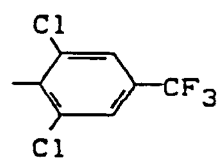 |
| H | $CF_3$ | $CH_3$ | 0 | 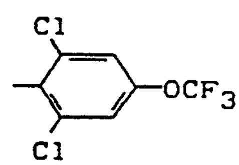 |
| H | $CF_3$ | $CH_3$ | 1 | 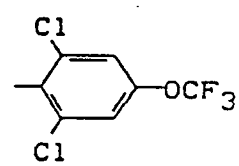 |
| H | $CF_3$ | $CH_3$ | 2 | 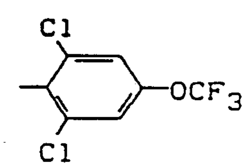 |

| $R^1$ | $R^2$ | $R^3$ | n | Ar |
|---|---|---|---|---|
| H | $CF_2Cl$ | $CH_3$ | 0 | 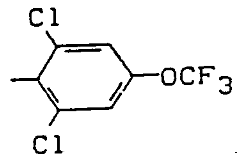 |
| H | $CF_2Cl$ | $CH_3$ | 1 | 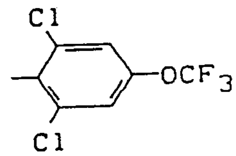 |
| H | $CF_2Cl$ | $CH_3$ | 2 | 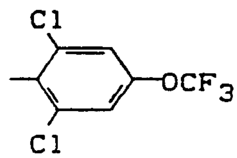 |
| H | $CFCl_2$ | $CH_3$ | 0 | 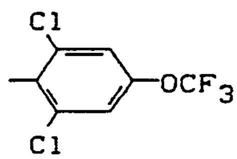 |
| H | $CFCl_2$ | $CH_3$ | 1 | 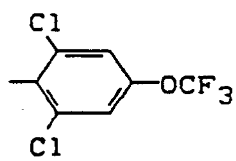 |
| H | $CFCl_2$ | $CH_3$ | 2 | 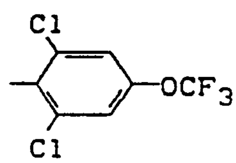 |
| $CF_3$ | $CF_3$ | $CF_3$ | 0 | 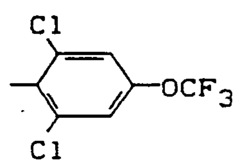 |
| H | $CF_3$ | $CF_3$ | 0 | 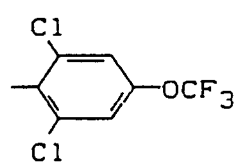 |

| $R^1$ | $R^2$ | $R^3$ | n | Ar |
|---|---|---|---|---|
| H | $CH_3$ | $CF_3$ | 0 | 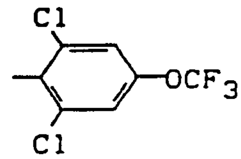 |
| $CF_3$ | $CF_3$ | $CF_3$ | 0 | 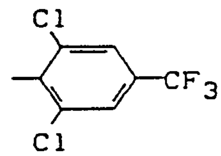 |
| H | $CF_3$ | $CF_3$ | 0 | 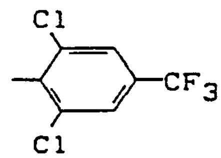 |
| $CH_3$ | $CF_3$ | $CF_3$ | 0 | 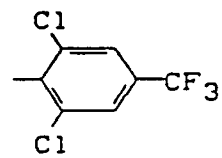 |
| H | $CH_3$ | $CF_3$ | 0 | 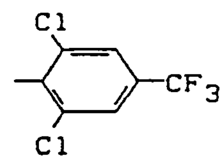 |

Verwendet man beispielsweise 2,6-Dichlor-4-trifluormethylphenylhydrazin Hydrochlorid und 1-Dimethylamino-2-trifluormethylthio-but-1-en-3-on als Ausgangsstoffe, so läßt sich der Reaktionsablauf des erfindungsgemäßen Verfahrens (a) durch das folgende Formelschema darstellen:

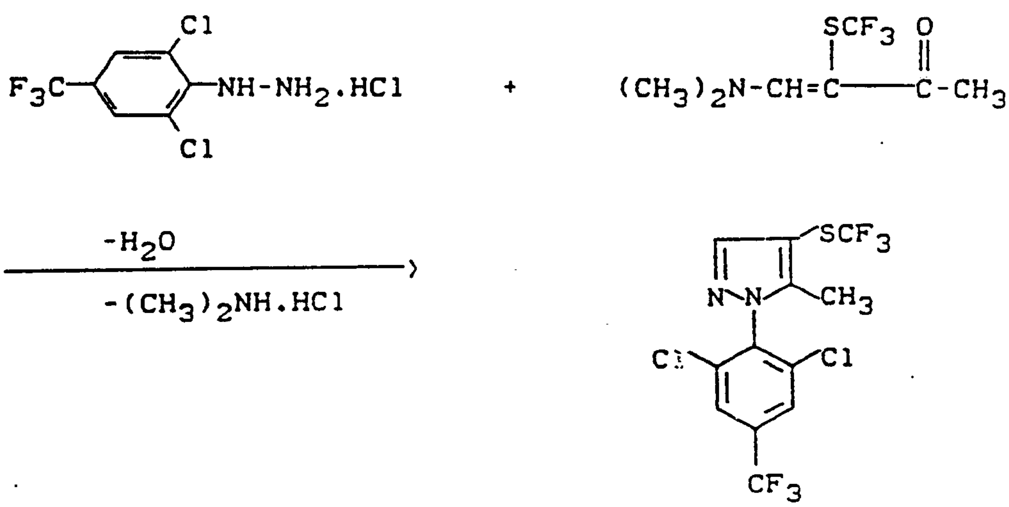

Verwendet man beispielsweise 5-Amino-1-(2,6-dichlor-4-trifluormethyl-phenyl)-3-methyl-4-trifluormethylthiopyrazol sowie t-Butylnitrit und Benzol als Ausgangsstoffe, so läßt sich der Reaktionsablauf des erfindungsgemäßen Verfahrens (b) durch das folgende Formelschema darstellen:

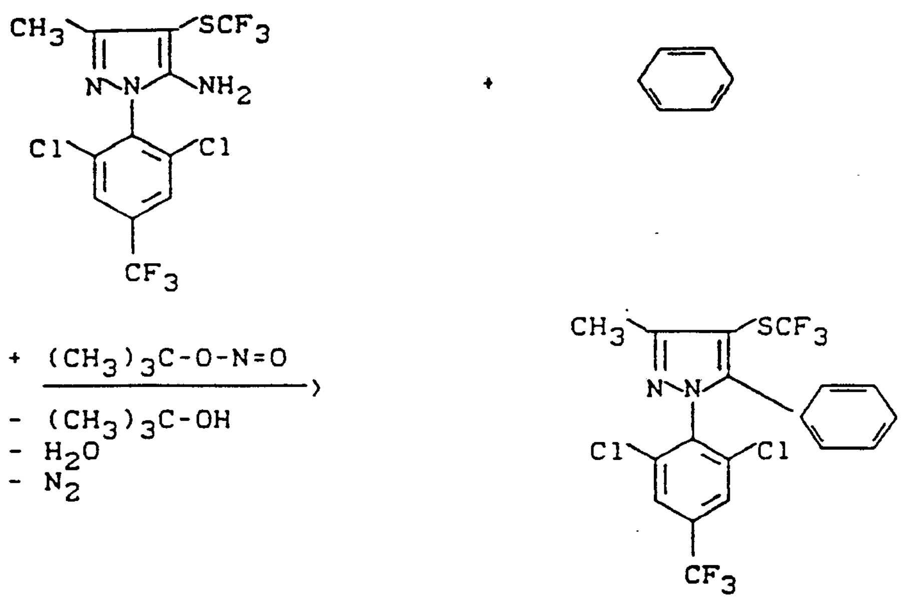


Verwendet man beispielsweise 1-(2,6-Dichlor-4-trifluormethyl-phenyl)-5-methyl-4-trifluormethylthiopyrazol als Ausgangsverbindung und m-Chlorperbenzoesäure als oxidationsmittel, so läßt sich der Reaktionsablauf des erfindungsgemäßen Verfahrens (c) durch das folgende Formelschema daratellen:

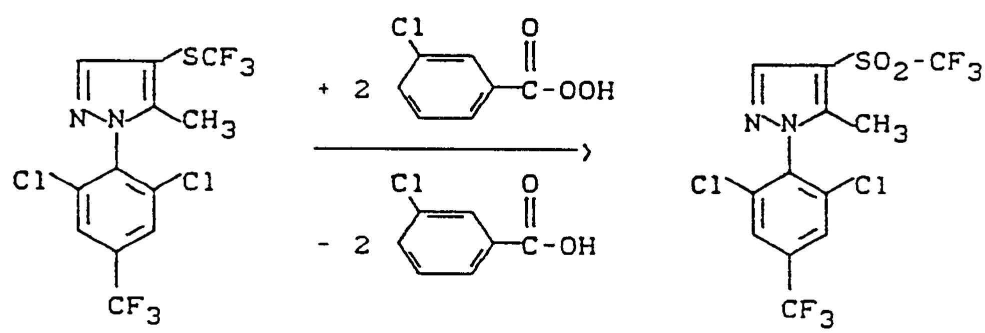


Die zur Durchführung des erfindungsgemäßen Verfahrens (a) als Ausgangsstoffe benötigten Arylhydrazine sind durch die Formel (II) allgemein definiert. In dieser Formel (II) steht Ar vorzugsweise für diejenigen Reste, die bereits im Zusammenhang mit der Beschreibung der erfindungsgemäßen Stoffe der Formel (I) als bevorzugt für diesen Substituenten genannt wurden.

Die Arylhydrazine der Formel (II) sind bekannt (vgl. z.B. US—PS 4 127 575; US—PS 3 609 158; DE—OS 2 558 399; DE—OS 3 402 308; DE—OS 3 408 727; J. Chem. Soc. C, *1971*, 167—174) oder lassen sich nach prinzipiell bekannten Verfahren in einfacher analoger Weise herstellen (vgl. Houben-Weyl "Methoden der organischen Chemie" Band X, 2 S.203, Thieme Verlag Stuttgart 1967), beispielsweise wenn man die allgemein bekannten Aniline bzw. Pyridylamine der Formel (VII),

$$Ar—NH_2 \qquad (VII)$$

in welcher

Ar die oben angegebene Bedeutung hat,

mit Natriumnitrit in Gegenwart einer Säure wie beispielsweise Schwefelsäure, und dann mit Zinn-II-Chlorid ebenfalls in Gegenwart einer Säure, wie beispielsweise Salzsäure bei Temperaturen zwischen −20°C und +80°C umsetzt oder indem man die ebenfalls allgemein bekannten Halogenaromaten der Formel (VIII),

$$Ar—Hal^1 \qquad (VIII)$$

in welcher

Ar die oben angegebene Bedeutung hat und

$Hal^1$ für Halogen, insbesondere für Fluor, Chlor oder Brom steht,
mit Hydrazinhydrat gegebenenfalls in Gegenwart eines Verdünnungsmittels wie beispielsweise Pyridin oder dioxan bei Temperaturen zwischen 0°C und +150°C umsetzt.

Die zur Durchführung des erfindungsgemäßen Verfahrens (a) weiterhin als Ausgangsstoffe benötigten 1,3-Diketone sind durch die Formel (IIIa) allgemein definiert. In dieser Formel (IIIa) stehen $R^1$. $R^2$ und $R^3$ vorzugsweise für die jenigen Reste, die bereits im Zusammenhang mit der Beschreibung der erfindungsgemäßen Stoffe der Formel (I) als bevorzugt für diese substituenten genannt wurden.

Die 1,3-Diketone der Formel (IIIa) sind bekannt (vgl. z.B. chem. Ber. *106*, 1418—1422 [1973]; J. org. Chem. *38*, 2809—2813 [1973]; J. org. Chem. *46*, 153—157 [1981]; J. org. Chem. *49*, 3494—3498 [1984]; DE—OS 2 138 728) oder lassen sich in Analogie zu bekannten Verfahren herstellen, beispielsweise, wenn man 1,3-Diketone der Formel (IX),

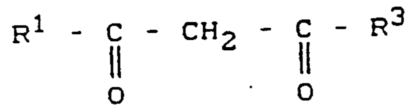

(IX)

in welcher
$R^1$ und $R^3$ die oben angegebene Bedeutung haben,
mit Sulfenylchloriden der Formel (X),

$$R^2—S—Cl \qquad (X)$$

in welcher
$R^2$ die oben angegebene Bedeutung hat,
gegebenenfalls in Gegenwart eines Verdünnungsmittels wie beispielsweise Toluol oder Dioxan und gegebenenfalls in Gegenwart eines Säurebindemittels wie beispielsweise Triethylamin bei Temperaturen zwischen 0°C und 50°C umsetzt;
oder wenn man 2-Halogen-1,3-diketone der Formel (XI)

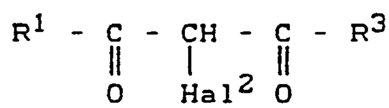

(XI)

in welcher
$R^1$ und $R^3$ die oben angegebene Bedeutung haben und
$Hal^2$ für Halogen, insbesondere für Chlor oder Brom steht,
mit Thiolen der Formel (XII),

$$R^2—SH \qquad (XII)$$

in welcher
$R^2$ die oben angegebene Bedeutung hat,
gegebenenfalls in Gegenwart eines Verdünnungsmittels wie beispielsweise Methanol oder Dimethylformamid und gegebenenfalls in Gegenwart eines Säurebindemittels wie beispielsweise Natriumhydroxid oder Natriummethylat bei Temperaturen zwischen 0°C und 80°C umsetzt.

Die 1,3-Diketone der Formel (IX), die Sulfenylchloride der Formel (X), die 2-Halogen-1,3-diketone der Formel (XI) und die Thiol der Formel (XII) sind allgemein bekannte Verbindungen der organischen Chemie.

Die zur Durchführung des erfindungsgemäßen Verfahrens (a) alternativ als Ausgangsstoffe benötigten α,β-ungesättigten Ketone sind durch die Formel (IIIb) allgemein definiert. In dieser Formel (IIIb) stehen $R^1$, $R^2$ und $R^3$ vorzugsweise für diejenigen Reste, die bereits im Zusammenhang mit der Beschreibung der erfindungsgemäßen Stoffe der Formel (I) als bevorzugt für diese Substituenten genannt wurden.

$A^1$ steht vorzugsweise für Methoxy, Ethoxy oder Dimethylamino.

Die α,β-ungesättigten Ketone der Formel (IIIb) sind größtenteils bekannt (vgl. z.B. J. org. Chem. *49*, 3494—3498 [1984]; Tetrahedron Letters, 3439—3442, *1967*, DE—OS 3 012 597) oder lassen sich in Analogie zu bekannten Verfahren herstellen, beispielsweise wenn man Ketone der Formel (XIII),

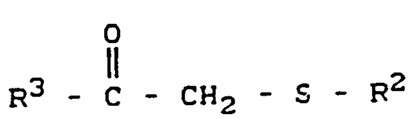

(XIII)

in welcher
$R^2$ und $R^3$ die oben angegebene Bedeutung haben,

mit Orthoestern bzw. Amidacetalen der Formel (XIV),

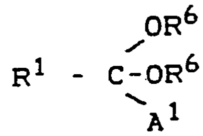

(XIV)

in welcher

$R^1$ und $A^1$ die oben angegebene Bedeutung haben und

$R^6$ für Alkyl, insbesondere für Methyl oder Ethyl steht,

gegebenenfalls in Gegenwart eines Verdünnungsmittels wie beispielsweise Toluol und gegebenenfalls in Gegenwart eines sauren Katalysators wie beispielsweise Bortrifluoridetherat bei Temperaturen zwischen 50°C und 150°C umsetzt.

Die Ketone der Formel (XIII) sind bekannt (vgl. z.B. Chem. Ber. *106*, 1418—1422 (1973); DE—OS 2 138 728; Zh. org. Khim. *8*, 1990—1991 (1972); US—PS 3 932 508, US—PS 3 937 738).

Die Orthoester bzw. Amidacetale der Formel (XIV) sind allgemein bekannte Verbindungen der organischen Chemie.

Die zur Durchführung des erfindungsgemäßen Verfahrens (b) als Ausgangsstoffe benötigen 5-Amino-1-aryl-pyrazole sind durch die Formel (IV) allgemein definiert. In dieser Formel (IV) stehen $R^1$, $R^2$ und Ar vorzugsweise für diejenigen Reste, die bereits im Zusammenhang mit der Beschreibung der erfindungsgemäßen Stoffe der Formel (I) als bevorzugt für diese Substituenten genannt wurden.

Die 5-Amino-1-aryl-pyrazole der Formel (IV) sind noch nicht bekannt. Sie sind jedoch Gegenstand der eigenen vorgängigen deutschen Patentanmeldungen P 3 402 308 (Le A 22 853) und DE—P 3 517 843 (Le A 23 753).

Man erhält sie, wenn man

4-Thiocyanato-5-aminopyrazole der allgemeinen Formel (XV),

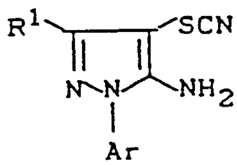

(XV)

in welcher

$R^1$ und Ar die oben angegebene Bedeutugn haben, oder

Bis-(pyrazolyl)-disulfide der Formel (XVI),

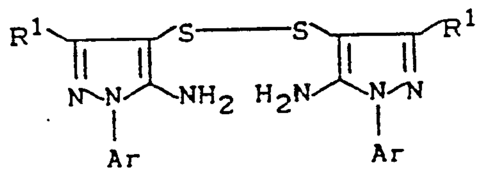

(XVI)

in welcher

$R^1$ und Ar die oben angegebene Bedeutung haben,

mit Halogeniden der Formel (XVII),

$$R^2—Hal^3 \quad (XVII)$$

in welcher

$R^2$ die oben angegebene Bedeutung hat und

$Hal^3$ für Halogen, insbesondere für Chlor, Brom oder Iod steht,

gegebenenfalls in Gegenwart eines Verdünnungsmittels wie beispielsweise Methanol oder Ethanol sowie gegebenenfalls in Gegenwart eines Reduktionsmittels wie beispielsweise Natriumborhydrid oder Natriumdithionit und gegebenenfalls in Gegenwart einer Base, wie beispielsweise Natriumhydroxid oder Kaliumcarbonat bei Temperaturen zwischen 20°C und 90°C umsetzt, oder wenn man

4-unsubstituierte 5-Amino-pyrazole der Formel (XVIII),

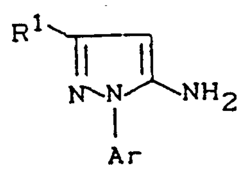

(XVIII)

in welcher

$R^1$ und Ar die oben angegebene Bedeutung haben,

mit Sulfenylhalogeniden der Formel (XIX),

$$R^2\text{—S—Hal}^4 \qquad \text{(XIX)}$$

in welcher

$R^2$ die oben angegebene Bedeutung hat und

$Hal^4$ für Halogen, insbesondere für Fluor, Chlor, Brom oder Iod steht,

gegebenenfalls in Gegenwart eines Verdünnungsmittels wie beispielsweise Dichlormethan und gegebenenfalls in Gegenwart eines Säurebindemittels wie beispielsweise Pyridin bei Temperaturen zwischen 0°C und 50°C umsetzt.

Die 4-Thiocyanato-5-aminopyrazole der Formel (XV) sind teilweise bekannt (vgl. z.B. Farmaco Ed. Sci. *38*, 274—282 [1983]. Man erhält sie beispielsweise, wenn man 4-unsubstituierte 5-Aminopyrazole der Formel (XVIII),

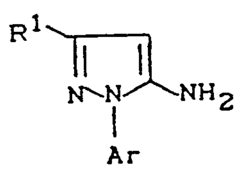

(XVIII)

in welcher

$R^1$ und Ar die oben angegebene Bedeutung haben,

mit Ammoniumthiocyanat in Gegenwart von Brom und Essigsäure bei Temperaturen zwischen −20°C und +20°C umsetzt.

Die Bis-(pyrazol)-disulfide der Formel (XVI) sind noch nicht bekannt. Man erhält sie, wenn man die oben beschriebenen 4-Thiocyanato-5-amino-pyrazole der Formel (XV) mit wässriger Salzsäure, gegebenenfalls in Gegenwart eines Verdünnungsmittels, wie beispielsweise Ethanol bei Temperaturen zwischen 20°C und 120°C umsetzt.

Die Halogenide der Formel (XVII) sind allgemein bekannte Verbindungen der organischen Chemie.

Die 4-unsubstituierten 5-Aminopyrazole der Formel (XVIII) sind teilweise bekannt (vgl. z.B. J. Org. Chem. *36*, 2972—2974 [1971] oder J. Heterocyclic Chemistry *7*, 345—349 [1970]; C.A. *62*; 13137c).

Men erhält sie beispielsweise, wenn man Arylhydrazine der Formel (II),

$$\text{Ar—NH—NH}_2 \qquad \text{(II)}$$

in welcher

Ar die oben angegebene Bedeutung hat,

mit Acrylnitrilderivaten der Formel (XX),

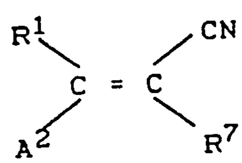

(XX)

in welcher

$R^1$ die oben angegebene Bedeutung hat,

$R^7$ für Wasserstoff oder Alkoxycarbonyl steht und

$A^2$ für Halogen, Hydroxy, Alkoxy, Amino oder Dialkylamino steht,

entweder zunächst in einer 1. Stufe, gegebenenfalls in Gegenwart eines Verdünnungsmittels wie beispielsweise Ethanol oder Eisessig und gegebenenfalls in Gegenwart eines Reaktionshilfsmittels wie beispielsweise Natriumacetat bei Temperaturen zwischen −20°C und +20°C umsetzt zu den Arylhydrazinderivaten der Formel (XXI),

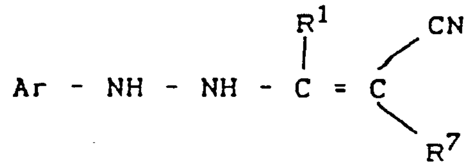

(XXI)

in welcher

Ar, $R^1$ und $R^7$ die oben angegebene Bedeutung haben,

und diese in einer 2. Stufe, gegebenenfalls in Gegenwart eines Verdünnungsmittels wie beispielsweise Ethylenglykolmonoethylether bei Temperaturen zwischen +50°C und +150°C cyclisiert, oder direkt in einem Reaktionsschritt ohne Isolierung der Zwischenstufe der Formel (XXI), gegebenenfalls in Gegenwart eines Verdünnungsmittels wie beispielsweise Ethylenglykolmonoethylether bei Temperaturen zwischen +50°C und +150°C direkt cyclisiert zu den 5-Aminopyrazolen der Formel (XXII),

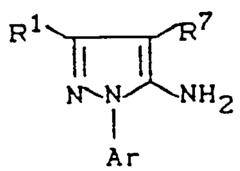

(XXII)

in welcher

$R^1$, $R^7$ und Ar die oben angegebene Bedeutung haben,

und in dem Fall, wo $R^7$ für Alkoxycarbonyl steht, die Verbindungen der Formel (XXII), in allgemein üblicher Art und Weise, gegebenenfalls in Gegenwart eines Verdünnungsmittels, wie beispielsweise Ethanol oder Isopropanol sowie gegebenenfalls in Gegenwart eines Katalysatos wie beispielsweise Bromwasserstoffsäure, bei Temperaturen zwischen 50°C und 150°C verseift und decarboxyliert.

Die Acrylnitrilderivate der Formel (XX) sind allgemein bekannte Verbindungen der organischen Chemie.

Die Sulfenylhalogenide der Formel (XIX) sind allgemein bekannte Verbindungen der organischen Chemie.

Die zur Durchführung des erfindungsgemäßen Verfahrens (b) weiterhin als Ausgangsstoffe benötigten Aromaten sind durch die Formel (V) allgemein definiert. In dieser Formel (V) steht $R^{3-1}$ vorzugsweise für einfach oder mehrfach gleich oder verschieden substituiertes Phenyl, wobei als Substituenten infrage kommen: Halogen, Cyano, Nitro, jeweils geradkettiges oder verzweigtes Alkyl, Alkoxy, Alkylthio, Alkylsulfinyl, Alkylsulfonyl, Halogenalkyl, Halogenalkoxy, Halogenalkylthio, Halogenalkylsulfinyl oder Halogenalkylsulfonyl mit jeweils 1 bis 4 Kohlenstoffatomen in den einzelnen Alkylteilen und gegebenenfalls 1 bis 9 gleichen oder verschiedenen Halogenatomen.

$R^{3-1}$ steht besonders bevorzugt für ein- bis dreifach gleich oder verschieden substituiertes Phenyl, wobei als Substituenten besonders bevorzugt sind: Fluor, Chlor, Brom, Iod, Cyano, Nitro, Methyl, Ethyl, Methoxy, Methylthio, Trifluormethyl, Methylsulfinyl, Methylsulfonyl, Trifluormethoxy, Trifluormethylthio, Trifluormethylsulfinyl oder Trifluormethylsulfonyl.

Die Aromaten der Formel (V) sind allgemein bekannte Verbindungen der organischen Chemie.

Die zur Durchführung des erfindungsgemäßen Verfahrens (b) weiterhin als Ausgangsstoffe benötigten Alkylnitrite sind Durch die Formel (VI) allgemein definiert. In dieser Formel (VI) steht $R^4$ vorzugsweise für n- oder i-Propyl, n-, i- oder t-Butyl, n- oder i-Hexyl sowie n- oder i-Pentyl. Besonders bevorzugt steht $R^4$ für t-Butyl.

Die Alkylnitrite der Formel (VI) sind ebenfalls allgemein bekannte Verbindungen der organischen Chemie.

Die zur Durchführung des erfindungsgemäßen Verfahrens (c) als Ausgangsstoffe benötigten 1-Aryl-pyrazole sind durch die Formel (Ia) allgemein definiert. In dieser Formel (Ia) stehen $R^1$, $R^2$, $R^3$ und Ar vorzugsweise für diejenigen Reste, die bereits im Zusammenhang mit der Beschreibung der erfindungsgemäßen Stoffe der Formel (I) als bevorzugt für diese Substituenten genannt wurden.

Die 1-Aryl-pyrazole der Formel (Ia) sind erfindungsgemäße Verbindungen und erhältlich mit Hilfe der erfindungsgemäßen Verfahren (a) oder (b).

Als Verdünnungsmittel zur Durchführung des erfindungsgemäßen Verfahrens (a) kommen inerte organische Lösungsmittel infrage. Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chlkoroform, Tetrachlorkohlenstoff, Ether, wie Diethylether, Dioxan, Tetrahydrofuran oder Ethylenglykoldimethyl- oder diethylether, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester, Sulfoxide, wie Dimethylsulfoxid oder Alkohole wie Methanol, Ethanol oder Propanol.

Das erfindungsgemäße Verfahren (a) wird gegebenenfalls in Gegenwart eines geeigneten Katalysators durchgeführt. Als solche kommen insbesondere anorganische Mineralsäuren wie beispielsweise Salzsäure oder Schwefelsäure infrage. Es ist auch möglich, die als Ausgangsstoffe infrage kommenden Arylhydrazine der Formel (II) in Form von entsprechenden Säureadditionssalzen, wie beispielsweise Hydrochloriden einzusetzen.

Die Reaktionstemperaturen können bei der Durchführung des erfindungsgemäßen Verfahrens (a) in einem größeren Bereich variiert werden. Im allgemeinen arbeitet man bei Temperaturen zwischen 0°C und 180°C, vorzugsweise bei Temperaturen zwischen 20°C und 120°C.

Zur Durchführung des erfindungsgemäßen Verfahrens (a) setzt man pro Mol an Arylhydrazin der Formel (II) bzw. an einem entsprechenden Hydrosalz im allgemeinen 0.5 bis 10.0 Mol an 1,3-Diketon der Formel (IIIa) bzw. an α,β-ungesättigten Keton der Formel (IIIb) und gegebenenfalls 0.01 bis 1.0 Mol an saurem Katalysator ein. Die Reaktionsdurchführung, Aufarbeitung und Isolierung der 1-Arylpyrazole der Formel (Ia) erfolgt nach allgemein üblichen Methoden.

Bei Verwendung von 1,3-Diketonen der Formel (IIIa), bei welchen der Substituent $R^1$ verschieden von dem Substituenten $R^3$ ist, erhält man in der Regel Isomerengemische aus Verbindungen der Formel (Ia)

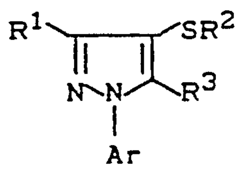

(Ia)

und Verbindungen der Formel (XXIII),

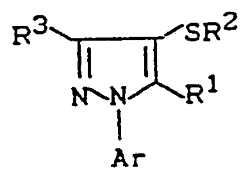

(XXII)

wobei

$R^1$, $R^2$, $R^3$ und Ar in beiden Formeln die oben angegebene Bedeutung haben.

Aus diesen Isomerengemischen lassen sich mit üblichen Trennverfahren (Destillation, Kristallisation, Chromatographie) die gewünschten Reaktionsprodukte der Formel (Ia) isolieren.

Als Verdünnungsmittel zur Durchführung des erfindungsgemäßen Verfahrens (b) kommen inerte organische Lösungsmittel infrage. Hierzu gehören insbesondere aliphatische gegebenenfalls halogenierte Kohlenwasserstoffe, wie beispielsweise Benzin, Petrolether, Hexan, Cyclohexan, Dichlormethan, Chloroform oder Tetrachlorkohlenstoff.

Mit besonderem Vorzug verwendet man die gleichzeitig als Reaktionskomponenten verwendeten Aromaten der Formel (V), falls diese bei der entsprechenden Reaktionstemperatur in flüssiger Form vorliegen, in entsprechendem Überschuß gleichzeitig als Verdünnungsmittel.

Die Reaktionstemperaturen können bei der Durchführung des erfindungsgemäßen Verfahrens (b) in einem größeren Bereich variiert werden. Im allgemeinen arbeitet man bei Temperaturen zwischen −20°C und +80°C, vorzugsweise bei Temperaturen zwischen 0°C und 50°C.

Zur Durchführung des erfindungsgemäßen Verfahrens (b) setzt man pro Mol an 5-Amino-1-arylpyrazol der Formel (IV) im allgemeinen 1.0 bis 3.0 Mol, vorzugsweise 1.0 bis 1.5 Mol an Alkylnitrit der Formel (VI) und 1.0 bis 30.0 Mol an Aromat der Formel (V) ein. Die Reaktionsdurchführung, Aufarbeitung und Isolierung der Reaktionsprodukte der Formel (Ib) erfolgt in Analogie zu bekannten Verfahren.

Als Oxidationsmitel zur Durchführung des erfindungsgemäßen Verfahrens (c) kommen alle üblichen zur Schwefeloxidation verwendbaren Oxidationsmittel infrage. Insbesondere geeignet sind Wasserstoffperoxid, organische Persäuren, wie beispielsweise Peressigsäure, m-Chlorperbenzoesäure, p-Nitroperbenzoesäure oder Luftsauerstoff.

Als Verdünnungsmittel zur Durchführung des erfindungsgemäßen Verfahrens (c) kommen ebenfalls inerte organische Lösungsmittel in Frage.

Vorzugsweise verwendet man Kohlenwasserstoffe, wie Benzin, Benzol, Toluol, Hexan oder Petrolether; chlorierte Kohlenwasserstoffe, wie Dichlormethan, 1,2-Dichlorethan, Chloroform, Tetrachlorkohlenstoff oder Chlorbenzol; Ether, wie Diethylether, Dioxan oder Tetrahydrofuran; Carbonsäuren, wie Essigsäure oder Propionsäure, oder dipolar aprotische Lösungsmittel, wie Acetonitril, Aceton, Essigsäureethylester oder Dimethylformamid.

Das erfindungsgemäße Verfahren (c) kann gegebenenfalls in Gegenwart eines Säurebindemittels durchgeführt werden. Als solche kommen alle üblicherweise verwendbaren organischen und anorganischen Säurebindemittel in Frage. Vorzugsweise verwendet man Erdalkali- oder Alkalimetallhydroxide, -acetate oder -carbonate, wie beispielsweise Calciumhydroxid, Natriumhydroxid, Natriumacetat oder Natriumcarbonat.

Das erfindungsgemäße Verfahren (c) kann gegebenenfalls in Gegenwart eines geeigneten Katalysators durchgeführt werden. Als solche kommen alle üblicherweise für derartige Schwefeloxidationen gebräuchlichen Metallsalz-Katalysatoren in Frage. Beispielhaft genannt sei in diesem Zusammenhang Ammoniummolybdat.

Die Reaktionstemperaturen können bei der Durchführung des erfindungsgemäßen Verfahrens (c) in einem größeren Bereich variiert werden. Im allgemeinen arbeitet man bei Temperaturen zwischen −20°C und +70°C, vorzugsweise bei Temperaturen zwischen 0°C und +50°C.

Zur Durchführung des erfindungsgemäßen Verfahrens (c) setzt man pro Mol an 1-Aryl-pyrazol der Formel (Ia) im allgemeinen 0.8 bis 1.2 Mol, vorzugsweise äquimolare Mengen Oxidationsmittel ein, wenn man die Oxidation des Schwefels auf der Sulfoxidstufe unterbrechen will. Zur Oxidation zum Sulfon setzt man pro Mol an 1-Aryl-pyrazol der Formel (Ia) im allgemeinen 1.8 bis 3.0 Mol, vorzugsweise doppelt molare Mengen an Oxidationsmittel ein. Die Reaktionsführung, Aufarbeitung und Isolierung der Endprodukte der Formel (Ic) erfolgt nach üblichen Verfahren.

Die Wirkstoffe eignen sich bei guter Pflanzenverträglichkeit und günstiger Warmblütertoxizität zur Bekämpfung von tierischen Schädlingen, insbesondere Insekten und Nematoden, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp.

Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp.,

Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp.

Aus der Ordnung der Arachnida z.B. Scorpio maurus, Latrodectus mactans.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten) wie Schildzecken, Lederzecken, Räubemilben, Laufmilben, Fliegen (stechend und leckend), parasitierende Fliegenlarven, Läuse, Haarlinge, Federlinge, Flöhe.

Sie sind gegen normalsensible und resistente Arten und Stämme, sowie gegen alle parasitierenden und nicht parasitierenden Entwicklungsstadien der Ektoparasiten wirksam.

Die erfindungsgemäßen Wirkstoffe zeichnen sich durch eine hohe insektizide Wirksamkeit aus. Sie lassen sich mit besonders gutem Erfolg gegen pflanzenschädigende Insekten, wie beispielsweise gegen die Larven der Meerrettichblattkäfer (Phaedon cochleariae) sowie die Raupen der Kohlschabe (Plutella maculipennis) einsetzen. Daneben eignen sie sich auch hervorragend zur Bekämpfung von Bodeninsekten und lassen sich beispielsweise zur Bekämpfung von Phorbia antiqua Maden oder von Diabrotica balteata Larven im Boden einsetzen.

Außerdem besitzen die erfindungsgemäßen Wirkstoffe eine hohe Wirkung gegen Hygiene- und Vorratsschädlinge und lassen sich beispielsweise zur Bekämpfung der Stubenfliege (Musca domestica), zur Bekämpfung des gemeinen Kornkäfers (Sitophilus granarius) oder zur Bekämpfung der deutschen Hausschabe (Blatella germanica) einsetzen. Darüber hinaus lassen sich die erfindungsgemäßen Wirkstoffe mit besonders gutem Erfolg zur Bekämpfung von parasitisch lebenden Warmblüterschädlingen wie beispielsweise gegen die Larven der Goldfliege (Lucilia cuprina) oder gegen Zecken (Boophilus microplus) einsetzen.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage; z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen im Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen

hergestellte Stoffe u.a.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemäß verwendbaren Wirkstoffe eignen sich auch zur Bekämpfung von Insekten, Milben, Zecken usw. auf dem Gebiet der Tierhaltung und Viehzucht, wobei durch die Bekämpfung der Schädlinge bessere Ergebnisse, z.B. höhere Milchleistungen, höheres Gewicht, schöneres Tierfell, längere Lebensdauer usw. erreicht werden können.

Die Anwendung der erfindungsgemäß verwendbaren Wirkstoffe geschieht auf diesem Gebiet in bekannter Weise wie durch orale Anwendung in Form von beispielsweise Tabletten, Kapseln, Tränken, Granulaten, durch dermale bzw. äußerliche Anwendung in Form beispielsweise des Tauchens (Dippen), Sprühens (Sprayen), Aufgießens (pour-on and spot-on) und des Einpuderns sowie durch parenterale Anwendung in Form beispielsweise der Injektion sowie ferner durch das "feed-through"-Verfahren. Daneben ist auch eine Anwendung als Formkörper (Halsband, Ohrmarke) möglich.

Die biologische Wirksamkeit der erfindungsgemäßen Verbindungen soll anhand der Beispiele A bis I erläutert werden, wobei die Publikation DE—A—2 839 270 als Stand der Technik angesehen wird.

## Herstellungsbeispiele

### Beispiel 1

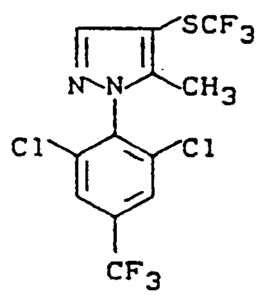

(Verfahren a)

Eine Mischung aus 29 g (0.1 Mol) 2,6-Dichlor-4-trifluormethylphenylhydrazin Hydrochlorid und 21 g (0.1 Mol) 1-Dimethylamino-2-trifluormethylthio-but-1-en-3-on in 150 ml Ethanol werden 16 Stunden bei 70°C bis 75°C gerührt. Zur Aufarbeitung engt man im Vakuum ein, nimmt den Rückstand in Chloroform auf, wäscht mit Wasser, trocknet über Natriumsulfat, engt im Vakuum ein und destilliert den Rückstand im Hochvakuum. Man erhält 26 g (61% der Theorie) an 1-(2,6-Dichlor-4-trifluormethyl-phenyl)-5-methyl-4-trifluormethylthio-pyrazol vom Siedepunkt 140°C bei 0.02 mbar und vom Schmelzpunkt 54°C—56°C.

Herstellung der Ausgangsverbindung:

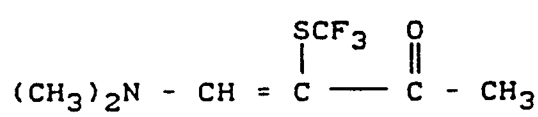

Ein Gemisch aus 16 g (0.1 Mol) Trifluormethylthioaceton (vgl. Chem. Ber. *106,* 1418—1422 [1973]) und 20 g (0.16 Mol) N,N-Dimethylformamid-dimethylacetel wird 3 Stunden auf 60°C erhitzt. Anschließend wird die Reaktionsmischung destilliert. Man erhält 18 g (84% der Theorie) an 1-Dimethylamino-2-trifluormethylthio-but-1-en-3-on vom Siedepunkt 110°C bei 6 mbar.

Beispiel 2

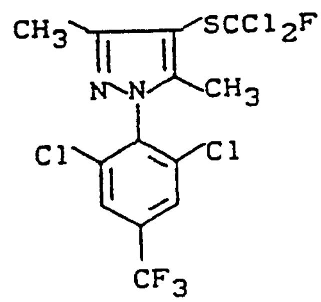

(Verfahren a)

24,5 g (0.1 Mol) 2,6-Dichlor-4-trifluormethyl-phenyl-hydrazin und 233 g (0.1 Mol) 3-Dichlorfluormethyl-thiopentan-2,4-dion in 200 ml Ethanol werden 24 Stunden unter Rückfluß erhitzt. Darauf setzt man 4 ml konzentrierte Schwefelsäure zu und erhitzt für weitere 4 Stunden auf Rückflußtemperatur. Zur Aufarbeitung engt man die Reaktionsmischung im Vakuum ein, nimmt den Rückstand in Dichlormethan auf, wäscht mit wässriger Natriumbicarbonatlösung, trocknet über Natriumsulfat und entfernt das Lösungsmittel im Vakuum. Man erhält 39,2 g (88% der Theorie) an 1-(2,6-Dichlor-4-trifluormethyl-phenyl)-3,5-dimethyl-4-dichlorfluormethylthio-pyrazol als Öl $^1$H-NMR ($CDCl_3$): δ = 7,78 ppm (2H·Aromat).

Beispiel 3

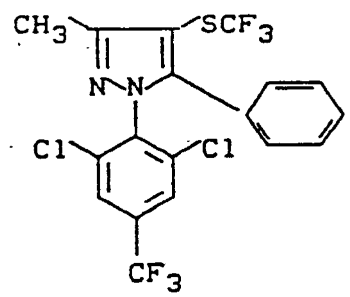

(Verfahren b)

Zu 4,1 g (0.01 Mol) 5-Amino-1-(2,6-dichlor-4-trifluormethyl-phenyl)-3-methyl-4-trifluormethylthio-pyrazol in 30 ml Benzol gibt man unter Rühren bei Raumtemperatur 2 ml (0.015 Mol) n-Pentylnitrit und rührt weitere 15 Stunden bei Raumtemperatur. Zur Aufarbeitung engt man im Vakuum ein und reinigt den Rückstand chromatographisch (Kieselgel; Laufmittel: Petrolether/Essigester 9:1). Man erhält 2,3 g (49% der Theorie) an 1-(2,6-Dichlor-4-trifluormethyl-phenyl)-3-methyl-5-phenyl-4-trifluormethylthio-pyrazol als Öl.

$^1$H-NMR ($CDCl_3$): δ = 7,34 ppm (5H·$C_6H_5$).

Herstellung der Ausgangsverbindung:

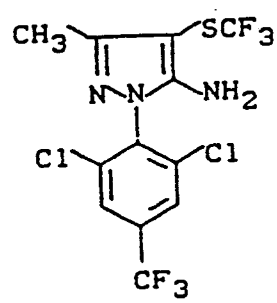

35,4 g (0,114 Mol) 5-Amino-1-(2,6-dichlor-4-trifluormethylphenyl)-3-methyl-pyrazol und 10 ml (0.125 Mol) wasserfreies Pyridin in 150 ml Dichlormethan werden bei 0°C bis 5°C unter Rühren tropfenweise mit 10,2 ml (0.12 Mol) Trifluormethansulfenylchlorid versetzt. Nach beendeter Zugabe rührt man weitere 30 Minuten bei Raumtemperatur, gibt 100 ml Dichlormethan zu und wäscht nacheinander mit verdünnter Salzsäure, Wasser, Natriumbicarbonatlösung und wässriger Kochsalzlösung, trocknet über Magnesiumsulfat und entfernt das Lösungsmittel im Vakuum. Man erhält 45,8 g (98% der Theorie) an 5-Amino-1-(2,6-dichlor-4-trifluormethylphenyl)-4-dichlorfluormethylsulfenyl-3-methyl-pyrazol vom Schmelzpunkt 131°C.

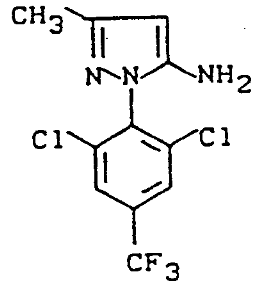

61,25 g (0.25 Mol) 2,6-Dichlor-4-trifluormethylphenyl-hydrazin und 21 g (0.25 Mol) Diacetonitril in 500 ml Ethanol werden 20 Stunden unter Rückfluß erhitzt. Zu der erkalteten Reaktionsmischung gibt man 4 ml konzentrierte Schwefelsäure und erhitzt für weitere 4 Stunden auf 60°C. Zur Aufarbeitung wird die Mischung im Vakuum eingedampft, der Rückstand in Chloroform aufgenommen und mit 25-prozentiger wässriger Ammoniaklösung alkalisch gestellt. Die organische Phase wird abgetrennt und die wässrige Phase mit Chloroform extrahiert. Die vereinigten organischen Phasen werden über Magnesiumsulfat getrocknet und im Vakuum vom Lösungsmittel befreit.

Man erhält 62 g (80% der Theorie) an 5-Amino-1-(2,6-dichlor-4-trifluormethylphenyl)-3-methyl-pyrazol als glasartige Substanz.

$^1$H-NMR ($CDCl_3$/TMS) δ = 2,23; 3,50; 5,49; 7,68 ppm.

Beispiel 4

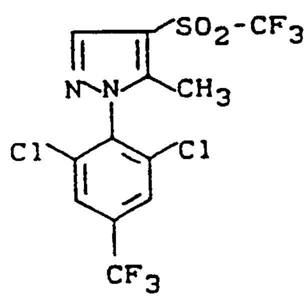

(Verfahren c)

7,9 g (0.02 Mol) 1-(2,6-Dichlor-4-trifluormethylphenyl)-5-methyl-4-trifluormethylthio-pyrazol in 60 ml Dichlormethan werden portionsweise mit 12 g (0.56 Mol) 80-prozentiger m-Chlorperbenzoesäure versetzt, 40 Stunden bei 25°C gerührt, filtriert, das Filtrat nacheinander mit gesättigter wässriger Natriumhydrogencarbonatlösung, gesättigter wässriger Natriumthiosulfatlösung, und nochmals mit gesättigter wässriger Natriumhydrogencarbonatlösung gewaschen, über Natriumsulfat getrocknet und im Vakuum eingeengt. Der Rückstand kristallisiert beim Verreiben mit Ligroin. Man erhält 3,5 g (41% der Theorie) an 1-(2,6-Dichlor-4-trifluormethyl-phenyl)-5-methyl-4-trifluormethylsulfonyl-pyrazol vom Schmelzpunkt 83°C—85°C.

In entsprechender Weise und gemäß den allgemeinen Angaben zur Herstellung erhält man die folgenden 1-Aryl-pyrazole der allgemeinen Formel (I):

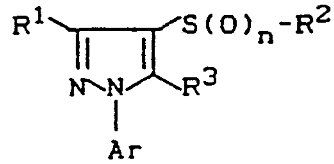

(I)

| Bsp. Nr. | $R^1$ | $-S(O)_n-R^2$ | $R^3$ | Ar | physikalische Daten |
|---|---|---|---|---|---|
| 5 | H | $-S(O)-CF_3$ | $CH_3$ | 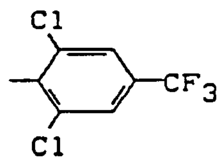 | |
| 6 | H | $-S-CF_2Cl$ | $CH_3$ | 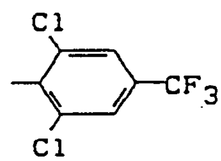 | Kp 180 °C/ 0.2 mbar |
| 7 | H | $-S-CF_3$ | 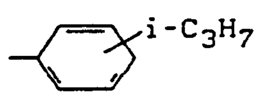 | 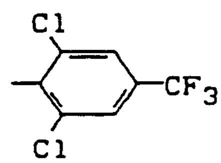 | $n_D^{20}$ 1.5275 |
| 8 | $CH_3$ | $-S-CCl_2F$ | $CH_3$ | 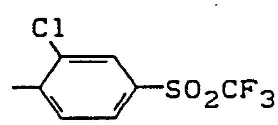 | Fp 69 °C-71 °C |
| 9 | $CH_3$ | $-S-CCl_2F$ | $CH_3$ | 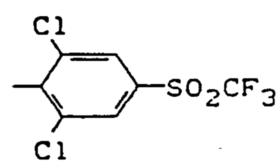 | Fp 160 °C |
| 10 | $CH_3$ | $-S-CCl_2F$ | $CH_3$ | 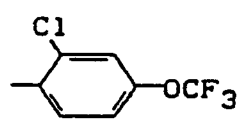 | Fp 49 °C-50 °C |

| Bsp. Nr. | $R^1$ | $-S(O)_n-R^2$ | $R^3$ | Ar | physikalische Daten |
|---|---|---|---|---|---|
| 11 | $CH_3$ | $-S-CF_3$ | 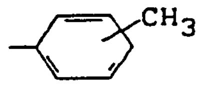 | 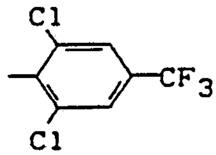 | $^1$H-NMR*): 2.51 |
| 12 | $CH_3$ | $-S-CF_2Cl$ | $CH_3$ | 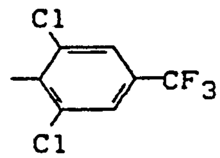 | $^1$H-NMR*): 2.21; 2.43 |
| 13 | $CH_3$ | $-S-CF_3$ | $CH_3$ | 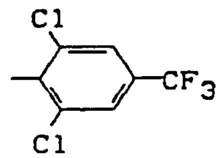 | $^1$H-NMR*): 2.19; 2.40 |
| 14 | H | $-S-CF_3$ | $CH_3$ | 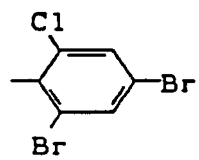 | Fp 55 °C-56 °C |
| 15 | H | $-S-CF_3$ | $CH_3$ | 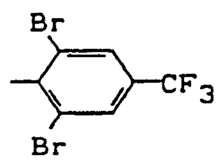 | Fp 80 °C-85 °C |
| 16 | H | $-S-CF_3$ | $CH_3$ | 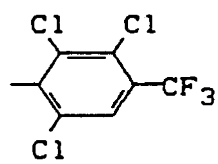 | Fp 80 °C-88 °C |
| 17 | $CH_3$ | $-S-CF_3$ | $CH_3$ | 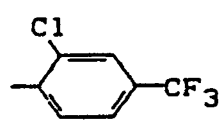 | $^1$H-NMR*): 2.24; 2.39 |
| 18 | $CH_3$ | $-S-CF_3$ | $CH_3$ | 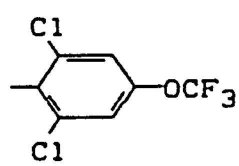 | $^1$H-NMR*): 2.18; 2.40 |

*) Die $^1$H-NMR-Spektren wurden in $CDCl_3$ mit Tetramethylsilan als innerem Standard aufgenommen. Angegeben ist die chemische Verschiebung als δ-Wert in ppm.

| Bsp. Nr. | $R^1$ | $-S(O)_n-R^2$ | $R^3$ | Ar | physikalische Daten |
|---|---|---|---|---|---|
| 19 | $CH_3$ | $-S-CF_3$ | $CH_3$ | 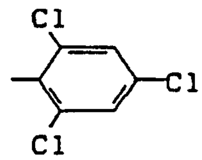 | $^1$H-NMR*): 2.17; 2.38 |
| 20 | H | $-S-CF_3$ | $CH_3$ | 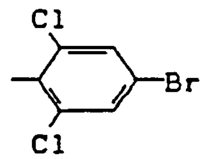 | Fp 47 °C-49 °C |
| 21 | H | $-S-CF_3$ | $i-C_3H_7$ | 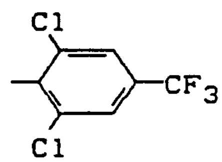 | Kp 140 °C/ 0.02 mbar |
| 22 | H | $-SCF_3$ | $CH_3$ | 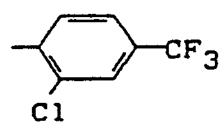 | Kp 180 °C/ 0.2mbar |
| 23 | H | $-SCF_3$ | $CH_3$ | 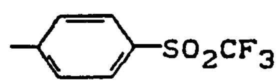 | Fp 47 °C-53 °C |
| 24 | H | $-SCF_3$ | $CH_3$ | 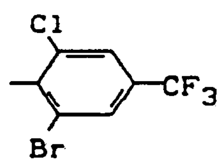 | Fp 53 °C-56 °C |
| 25 | H | $-SCF_3$ | $CH_3$ | 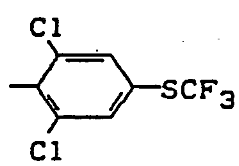 | Kp 180 °C/ 0.2 mbar |
| 26 | H | $-SCClF_2$ | $CH_3$ | 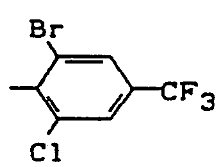 | Fp 59 °C-64 °C |
| 27 | H | $-SCClF_2$ | $CH_3$ | 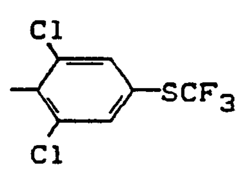 | Kp 170 °C/ 0.2 mbar |

*) Die $^1$H-NMR-Spektren wurden in $CDCl_3$ mit Tetramethylsilan als innerem Standard aufgenommen. Angegeben ist die chemische Verschiebung als δ-Wert in ppm.

| Bsp. Nr. | $R^1$ | $-S(O)_n-R^2$ | $R^3$ | Ar | physikalische Daten |
|---|---|---|---|---|---|
| 28 | H | $-SCClF_2$ | $CH_3$ | 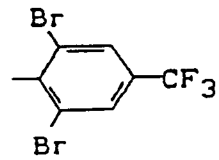 | Fp 79 °C-84 °C |
| 29 | H | $-SCClF_2$ | $CH_3$ | 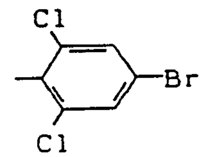 | Fp 63 °C-65 °C |
| 30 | H | $-SCClF_2$ | $CH_3$ | 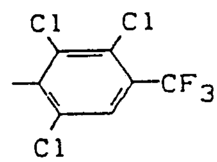 | Fp 64 °C-72 °C |
| 31 | H | $-SCClF_2$ | $CH_3$ | 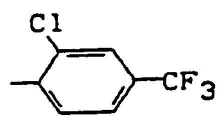 | Kp 170 °C/ 0.2 mbar |

### Beispiel A

Phaedon-Larven-Test

Lösungsmittel: 7 Gewichtsteile Dimethylformamid

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleacea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100%, daß alle Käferlarven abgetötet wurden.

Bei diesem Test zeigen z.B. die folgenden Verbindungen der Herstellungsbeispiele überlegene Wirksamkeit gegenüber dem Stand der Technik: 1, 2, 4, 8, 9, 10.

### Beispiel B

Plutella-Test

Lösungsmittel: 7 Gewichtsteile Dimethylformamid

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind.

Nach der gewünschten Zeit wird die Abtötung in % bestimmt. Dabei bedeuten 100%, daß alle Raupen abgetötet wurden; 0% bedeutet, daß keine Raupen abgetötet wurden.

Bei diesem Test zeigen z.B. die folgenden Verbindungen der Herstellungsbeispiele überlegene Wirksamkeit gegenüber dem Stand der Technik: 1, 2, 4, 8, 10.

### Beispiel C

Grenzkonzentrations-Test/Bodeninsekten

Testinsekt: Phorbia antiqua-Maden (im Boden)

Lösungsmittel: 3 Gewichtsteile Aceton

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den Boden in Töpfe und läßt diese bei Raumtemperatur stehen.

Nach 24 Stunden werden die Testtiere in den behandelten Boden gegeben und nach weiteren 2 bis 7 Tagen wird der Wirkungsgrad des Wirkstoffs durch Auszählen der toten und lebenden Testinsekten in % bestimmt. Der Wirkungsgrad ist 100%, wenn alle Testinsekten abgetötet worden sind, er ist 0%, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik: 1, 2, 4.

## Beispiel D

Grenzkonzentration/Bodeninsekten

Testinsekt: Diabrotica balteata — Larven im Boden

Lösungsmittel: 3 Gewichtsteile Aceton

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration. Die Wirkstoffzubereitung wird innig mit dem Boden vermischt. Dabei spielt die Konzentration des Wirkstoffes in der Zubereitung praktisch keine Rolle, entsprechend ist allein die Wirkstoffgewichtsmenge pro Volumeneinheit Boden, welche in ppm (mg/1) angegeben wird. Man füllt den Boden in 0,5 l Töpfe und läßt diese bei 20°C stehen.

Sofort nach dem Ansatz werden je Topf 6 vorgekeimte Maiskörner gelegt. Nach 2 Tagen werden in den behandelten Boden die entsprechenden Testinsekten gesetzt. Nach weiteren 7 Tagen wird der Wirkungsgrad des Wirkstoffes durch Auszählen der toten und lebenden Testinsekten in % bestimmt. Der Wirkungsgrad ist 100%, wenn alle Testinsekten abgetötet worden sind, er ist 0%, wenn noch genau so viele Testinskten leben wie in der unbehandelten Kontrolle.

Bei diesem Test zeigen z.B. die folgenden Verbindungen der Herstellungsbeispiele überlegene Wirkung gegenüber dem Stand der Technik: 1, 2, 4.

## Beispiel E

$LD_{100}$-Test

Testtiere: Sitophilus granarius

Zahl der Testtiere: 25

Lösungsmittel: Aceton

2 Gewichtsteile Wirkstoff werden in 1000 Volumenteilen Lösungsmittel aufgenommen. Die so erhaltene Lösung wird mit weiterem Lösungsmittel auf die gewünschten Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^2$ Filterpapier verschieden hoch. Anschließend gibt man die angegebene Anzahl der Testtiere in die Petrischale und bedeckt sie mit einem Glasdeckel.

Der Zustand der Testtiere wird 3 Tage nach Ansetzen der Versuche kontrolliert. Bestimmt wird die Abtötung in %. Dabei bedeutet 100%, daß alle Testtiere abgetöet wurden; 0% bedeutet, daß keine Testtiere abgetötet wurden.

Bei diesem Test zeigen z.B. die folgenden Verbindungen der Herstellungsbeispiele überlegene Wirkung gegenüber dem Stand der Technik: 2, 4, 10.

## Beispiel F

$LD_{100}$-Test

Testtiere: Blatella germanica

Lösungsmittel: Aceton

2 Gewichtsteile Wirkstoff werden in 1000 Volumenteilen Lösungsmittel aufgenommen. Die so erhaltene Lösung wird mit weiterem Lösungsmittel auf die gewünschten Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^2$ Filterpapier verschieden hoch. Anschließend gibt man etwa 20 Testtiere in die Petrischale und bedeckt sie mit einem Glasdeckel.

Der Zustand der Testtiere wird 3 Tage nach Ansetzen der Versuche kontrolliert. Bestimmt wird die Abtötung in %.

Bei diesem Test zeigen z.B. die folgenden Verbindungen der Herstellungsbeispiele überlegene Wirkung gegenüber dem Stand der Technik: 1 und 2.

Beispiel G

$LT_{100}$-Test für Dipteren

Testtiere: Musca domestica

Zahl der Testtiere: 25

Lösungsmittel: Aceton

2 Gewichtsteile Wirkstoff werden in 1000 Volumenteilen Lösungsmittel aufgenommen. Die so erhaltene Lösung wird mit weiterem Lösungsmittel auf die gewünschten geringeren Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^2$ Filterpapier verschieden hoch. Anschließend gibt man die angegebene Anzahl der Testtiere in die Petrischale und bedeckt sie mit einem Glasdeckel.

Der Zustand der Testtiere wird laufend kontrolliert. Es wird diejenige Zeit ermittelt, welche für einen 100%igen knock down-Effekt notwendig ist.

Bei diesem Test zeigen z.B. die folgenden Verbindungen der Herstellungsbeispiele überlegene Wirkung gegenüber dem Stand der Technik: 1, 2, 10.

Beispiel H

Test mit Boophilus microplus resistent/OP-resistenter Biarra-Stamm

Lösungsmittel: 35 Gewichtsteile Ethylenglykolmonomethylether
35 Gewichtsteile Nonylphenolpolyglykolether

Zur Herstellung einer zweckmäßigen Wirkstoffzubereitung vermischt man drei Gewichtsteile Wirkstoff mit sieben Gewichtsteilen des oben angegebenen Lösungsmittel-Gemisches und verdünnt das so erhaltene Konzentrat mit Wasser auf die gewünschte Konzentration.

10 adulte Boophilus microplus res. werden in die zu testende Wirkstoffzubereitung 1 Minute getaucht. Nach Überführung in Plastikbecher und Aufbewahrung in einem klimatisierten Raum wird der Abtötungsgrad bestimmt.

Bei diesem Test zeigt z.B. die Verbindung 2 der Herstellungsbeispiele überlegene Wirkung gegenüber dem Stand der Technik.

Beispiel I

Test mit Lucilia cuprina res.-Larven (OP-res. Goondiwindi-Stamm)

Emulgator: 35 Gewichtsteile Ethylenglykolmonomethylether
35 Gewichtsteile Nonylphenolpolyglykolether

Zur Herstellung einer zweckmäßigen Wirkstoffzubereitung vermischt man drei Gewichtsteile Wirkstoff mit sieben Gewichtsteilen des oben angegebenen Lösungsmittel-Gemisches und verdünnt das so erhaltene Konzentrat mit Wasser auf die jeweils gewünschte Konzentration.

Etwa 20 Lucilia cuprina res.-Larven werden in ein Teströhrchen gebracht, welches ca. 1 $cm^3$ Pferdefleisch und 0,5 ml der Wirkstoffzubereitung enthält. Nach 24 Stunden wird der Abtötungsgrad bestimmt.

Bei diesem Test zeigt z.B. die Verbindung 1 der Herstellungsbeispiele überlegene Wirkung gegenüber dem Stand der Technik.

**Patentansprüche**

1. 1-Aryl-pyrazole der allgemeinen Formel (I)

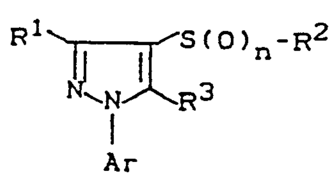

(I)

in welcher

$R^1$ für Wasserstoff, oder für jeweils geradkettiges oder verzweigtes Alkyl oder Halogenalkyl mit 1 bis 4 Kohlenstoffatomen und gegebenenfalls 1 bis 9 gleichen oder verschiedenen Halogenatomen steht,

$R^2$ für jeweils geradkettiges oder verzweigtes Alkyl, Alkenyl, oder Alkinyl mit jeweils bis zu 8 Kohlenstoffatomen, für Cycloalkyl mit 3 bis 7 Kohlenstoffatomen, für jeweils geradkettiges oder verzweigtes Halogenalkyl oder Halogenalkenyl mit jeweils bis zu 8 Kohlenstoffatomen und bis zu 17 gleichen oder verschiedenen Halogenatomen, für jeweils geradkettiges oder verzweigtes Alkoxyalkyl,

Alkylthioalkyl, Alkylsulfinylalkyl oder Alkylsulfonylalkyl mit jeweils 1 bis 6 Kohlenstoffatomen in den einzelnen Alkylteilen oder für jeweils im Phenylteil gegebenenfalls einfach oder mehrfach, gleich oder verschieden substituiertes Phenylalkyl oder Phenyl mit gegebenenfalls 1 bis 4 Kohlenstoffatomen im geradkettigen oder verzweigten Alkylteil steht, wobei als Substituenten im Phenylteil infrage kommen: Halogen, Cyano, Nitro, jeweils geradkettiges oder verzweigtes Alkyl, Alkoxy, Alkylthio, Alkylsulfinyl, Alkylsulfonyl, Halogenalkyl, Halogenalkoxy, Halogenalkylthio, Halogenalkylsulfinyl oder Halogenalkylsulfonyl mit jewils 1 bis 4 Kohlenstoffatomen in den einzelnen Alkylteilen und gegebenenfalls 1 bis 9 gleichen oder verschiedenen Halogenatomen,

$R^3$ für jeweils geradkettiges oder verzweigtes Alkyl oder Halogenalkyl mit jeweils 1 bis 6 Kohlenstoffatomen und gegebenenfalls 1 bis 12 gleichen oder verschiedenen Halogenatomen, für Cycloalkyl mit 3 bis 7 Kohlenstoffatomen oder für gegebenenfalls einfach oder mehrfach, gleich oder verschieden substituiertes Phenyl steht, wobei als Phenylsubstituenten die bei $R^2$ genannten infrage kommen,

Ar für einfach oder mehrfach, gleich oder verschieden substituiertes Phenyl mit Ausnahme des 4-Nitrophenyl-sowie des 2,4-Dinitrophenylrestes steht, wobei als Substituenten jeweils in Frage kommen: Cyano, Nitro, Halogen, jeweils geradkettiges oder verzweigtes Alkyl, Alkoxy oder Alkoxycarbonyl mit jeweils 1 bis 4 Kohlenstoffatomen, außerdem jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen oder ein Rest $—S(O)_p—R^5$, wobei

$R^5$ für Amino sowie für jeweils geradkettiges oder verzweigtes Alkyl, Alkylamino, Dialkylamino oder Halogenalkyl mit jeweils 1 bis 4 Kohlenstoffatomen in den einzelnen Alkylteilen und im Fall des Halogenalkyl mit 1 bis 9 gleichen oder verschiedenen Halogenatomen steht,

p für eine Zahl 0, 1 oder 2 steht und

n für eine Zahl 0, 1 oder 2 steht.

2. 1-Aryl-pyrazole der Formel (I) gemäß Anspruch 1, dadurch gekennzeichnet, daß

$R^1$ für Wasserstoff, Methyl, Ethyl, n- oder i-Propyl oder Trifluormethyl steht,

$R^2$ für Methyl, Ethyl, n- oder i-Propyl, n-, i-, s- oder t-Butyl, n- oder i-Pentyl, n- oder i-Hexyl, Allyl, n- oder i-Butenyl, n- oder i-Pentenyl, Propargyl, n- oder i-Butinyl, Cyclopropyl, Cyclobutyl, Cyclopentyl, Cyclohexyl, Chlormethyl, Difluormethyl, Difluorchlormethyl, Fluordichlormethyl, Trifluormethyl, Pentafluorethyl, Pentachlorethyl, Fluortetrachlorethyl, Difluortrichlorethyl, Trifluordichlorethyl, Tetrafluorchlorethyl, Heptafluorpropyl, Chlorethyl, Bromethyl, Chlorpropyl, Brompropyl, Dichlormethyl, Chlorfluormethyl, Trichlormethyl, Dichlormethyl, Trifluorethyl, Trifluorchlorethyl, Tetrafluorethyl, Difluorchlorethyl, Fluordibrommethyl, Difluorbrommethyl, Fluorchlorbrommethyl, Chloralkyl, Fluorallyl, Chlorbutenyl, Fluorbutenyl, Dichlorallyl, Fluorchlorallyl, Difluorallyl, Bromallyl, für Methoxymethyl, Methoxyethyl, Methoxypropyl, Ethoxymethyl, Ethoxyethyl, Methylthiomethyl, Methylsulfinylmethyl, Methylsulfonylethyl, Methylthioethyl, Methylthiopropyl, Methylsulfinylethyl, Methylsufonylmethyl oder für jeweils gegebenenfalls einfach bis dreifach, gleich oder verschieden substituiertes Phenyl, Benzyl oder Phenylethyl steht, wobei als Phenylsubstituenten in Frage kommen: Fluor, Chlor, Brom, Iod, Cyano, Nitro, Methyl, Ethyl, Methoxy, Methylthio, Trifluormethyl, Methylsulfinyl, Methylsulfonyl, Trifluormethoxy, Trifluormethylthio, Trifluormethylsulfinyl oder Trifluormethylsulfonyl,

$R^3$ für Methyl, Ethyl, n- oder i-Propyl, n-, i-, s- oder t-Butyl, Cyclopropyl, Cyclobutyl, Cyclopentyl, Cyclohexyl, für Chlormethyl, Dichlormethyl, Trichlormethyl, Brommethyl, Dibrommethyl, Fluormethyl, Difluormethyl, Trifluormethyl, Fluordichlormethyl, Difluorchlormethyl oder für gegebenenfalls ein- bis dreifach, gleich oder verschieden substituiertes Phenyl steht, wobei als Phenylsubstituenten die bei $R^2$ genannten infrage kommen,

Ar für ein- bis fünffach, gleich oder verschieden substituiertes Phenyl mit Ausnahme des 4-Nitrophenyl-sowie des 2,4-Dinitrophenylrestes steht, wobei als Substituenten jeweils in Frage kommen: Cyano, Nitro, Fluor, Chlor, Brom, Iod, Methyl, Ethyl, n- und i-Propyl, n-, i-, s- und t-Butyl, Methoxy, Ethoxy, Methoxycarbonyl, Ethoxycarbonyl, Trifluormethyl, Trichlormethyl, Dichlorfluormethyl, Difluorchlormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder ein Rest $—S(O)_p—R^5$, wobei

$R^5$ für Amino, Methylamino, Ethylamino, Dimethylamino, Diethylamino, Fluordichlormethyl, Difluorchlormethyl, Tetrafluorethyl, Trifluorchlorethyl, Trifluormethyl, Methyl oder Ethyl steht,

p für eine Zahl 0, 1 oder 2 steht und

n für eine Zahl 0, 1 oder 2 steht.

3. Verfahren zur Herstellung von 1-Aryl-pyrazolen der allgemeinen Formel (I)

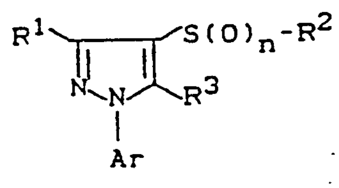

(I)

in welcher

$R^1$ für Wasserstoff, oder für jeweils geradkettiges oder verzweigtes Alkyl oder Halogenalkyl mit 1 bis 4 Kohlenstoffatomen und gegebenenfalls 1 bis 9 gleichen oder verschiedenen Halogenatomen steht,

$R^2$ für jeweils geradkettiges oder verzweigtes Alkyl, Alkenyl, oder Alkinyl mit jeweils bis zu 8 Kohlenstoffatomen, für Cycloalkyl mit 3 bis 7 Kohlenstoffatomen, für jeweils geradkettiges oder verzweigtes Halogenalkyl oder Halogenalkenyl mit jeweils biz zu 8 Kohlenstoffatomen und bis zu 17 gleichen oder verschiedenen Halogenatomen, für jeweils geradkettiges oder verzweigtes Alkoxyalkyl, Alkylthioalkyl, Alkylsulfinylalkyl oder Alkylsulfonylalkyl mit jeweils 1 bis 6 Kohlenstoffatomen in den einzelnen Alkylteilen oder für jeweils im Phenylteil gegebenenfalls einfach oder mehrfach, gleich oder verschieden substituiertes Phenylalkyl oder Phenyl mit gegebenenfalls 1 bis 4 Kohlenstoffatomen im geradkettigen oder verzweigten Alkylteil steht, wobei als Substituenten im Phenylteil infrage kommen: Halogen, Cyano, Nitro, jeweils geradkettiges oder verzweigtes Alkyl, Alkoxy, Alkylthio, Alkylsulfinyl, Alkylsulfonyl, Halogenalkyl, Halogenalkoxy, Halogenalkylthio, Halogenalkylsulfinyl oder Halogenalkylsulfonyl mit jewils 1 bis 4 Kohlenstoffatomen in dem einzelnen Alkylteilen und gegebenenfalls 1 bis 9 gleichen oder verschiedenen Halogenatomen,

$R^3$ für jeweils geradkettiges oder verzweigtes Alkyl oder Halogenalkyl mit jeweils 1 bis 6 Kohlenstoffatomen und gegebenenfalls 1 bis 12 gleichen oder verschiedenen Halogenatomen, für Cycloalkyl mit 3 bis 7 Kohlenstoffatomen oder für gegebenenfalls einfach oder mehrfach, gleich oder verschieden substituiertes Phenyl steht, wobei als Phenylsubstituenten die bei $R^2$ genannten infrage kommen,

Ar für einfach oder mehrfach, gleich oder verschieden substituiertes Phenyl mit Ausnahme des 4-Nitrophenyl-sowie des 2,4-Dinitrophenylrestes steht, wobei als Substituenten jeweils in Frage kommen: Cyano, Nitro, Halogen, jeweils geradkettiges oder verzweigtes Alkyl, Alkoxy oder Alkoxycarbonyl mit jeweils 1 bis 4 Kohlenstoffatomen, außerdem jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen oder ein Rest $—S(O)_p—R^5$, wobei

$R^5$ für Amino sowie für jeweils geradkettiges oder verzweigtes Alkyl, Alkylamino, Dialkylamino oder Halogenalkyl mit jeweils 1 bis 4 Kohlenstoffatomen in den einzelnen Alkylteilen und im Fall des Halogenalkyl mit 1 bis 9 gleichen oder verschiedenen Halogenatomen steht,

p für eine Zahl 0, 1 oder 2 steht und

n für eine Zahl 0, 1 oder 2 steht,

dadurch gekennzeichnet, daß man zum Erhalt der 1-Aryl-pyrazole der Formel (Ia),

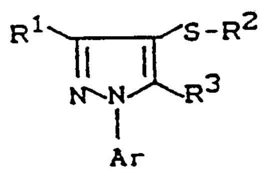

(Ia)

in welcher

$R^1$, $R^2$, $R^3$ und Ar die oben angegebene Bedeutung haben,

a) Arylhydrazine der Formel (II),

$$AR—NH—NH_2 \qquad (II)$$

in welcher

Ar die oben angegebene Bedeutung hat, oder deren Hydrosalze mit 1,3-Diketonen der Formel (IIIa)

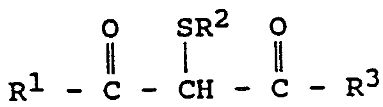

(IIIa)

in welcher

$R^1$, $R^2$ und $R^3$ die oben angegebene Bedeutung haben, oder alternativ mit αβ-ungesättigten Ketonen der Formel (IIIb),

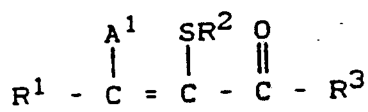

(IIIb)

in welcher

$R^1$, $R^2$ und $R^3$ die oben angegebene Bedeutung haben und

$A^1$ für eine Abgangsgruppe wie beispielsweise Alkoxy oder Dialkylamino steht,

gegebenenfalls in Gegenwart eines Verdünnungsmittels und gegebenenfalls in Gegenwart eines Katalysators umsetzt, oder daß man zum Erhalt der 1-Arylpyrazole der Formel (Ib),

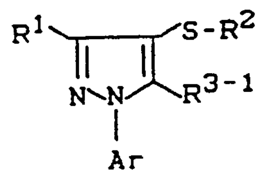

(Ib)

in welcher

$R^1$, $R^2$ und Ar die oben angegebene Bedeutung haben und

$R^{3-1}$ für gegebenenfalls substituiertes Aryl steht,

b) 5-Amino-1-aryl-pyrazole der Formel (IV),

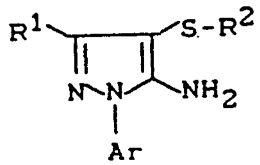

(IV)

mit Aromaten der Formel (V),

$$R^{3-1}—H \qquad (V)$$

in welcher

$R^{3-1}$ die oben angegebene Bedeutung hat,

in Gegenwart eines Alkylnitrits der Formel (VI),

$$R^4—O—N=O \qquad (VI)$$

in welcher

$R^4$ für Alkyl steht

und gegebenenfalls in Gegenwart eines Verdünnungsmittels umsetzt, oder daß man zum Erhalt der 4-Sulfinyl- und 4-Sulfonyl-1-aryl-pyrazole der Formel (Ic),

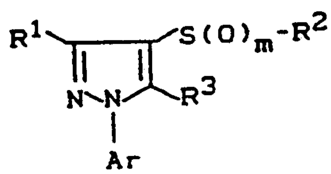

(Ic)

in welcher

$R^1$, $R^2$, $R^3$ und Ar die oben angegebene Bedeutung haben und

m für eine Zahl 1 oder 2 steht,

c) die nach Verfahren (a) oder (b) erhältlichen 1-Arylpyrazole der Formel (Ia),

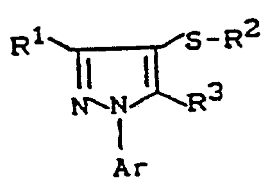

(Ia)

in welcher

$R^1$, $R^2$, $R^3$ und Ar die oben angegebene Bedeutung haben,

mit Oxidationsmitteln gegebenenfalls in Gegenwart eines Verdünnungsmittels und gegebenenfalls in Gegenwart eines Katalysators oxidiert.

4. Schädlingsbekämpfungsmittel, gekennzeichnet durch einen Gehalt an mindestens einem 1-Arylpyrazol der Formel (I).

5. Insektizide und nematizide Mittel, gekennzeichnet durch einen Gehalt an mindestens einem 1-Arylpyrazol der Formel (I).

6. Verfahren zur Bekämpfung von Insekten und/oder Nematoden, dadurch gekennzeichnet, daß man 1-Aryl-pyrazole der Formel (I) auf Insekten und/oder Nematoden und/oder deren Lebensraum einwirken läßt.

7. Verwendung von 1-Aryl-pyrazolen der Formel (I) zur Bekämpfung von Insekten und/oder Nematoden.

8. Verfahren zur Herstellung von Schädlingsbekämpfungsmitteln, dadurch gekennzeichnet, daß man 1-Aryl-pyrazole der Formel (I) mit Streckmitteln und/oder oberflächenaktiven Mitteln vermischt.

**Revendications**

1. 1-Arylpyrazoles de formule générale (I)

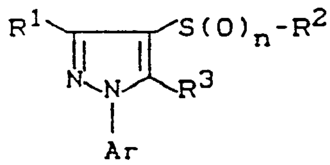

(I)

dans laquelle

$R^1$ représente l'hydrogène ou un groupe alkyle ou halogénalkyle à chaîne droite ou ramifiée ayant 1 à 4 atomes de carbone et le cas échéant 1 à 9 atomes d'halogènes identiques ou différents,

$R^2$ est un groupe alkyle, alcényle, ou alcinyle à chaîne droite ou ramifiée ayant chacun jusqu'à 8 atomes de carbone, un groupe cycloalkyle de 3 à 7 atomes de carbone, un groupe halogénalkyle ou halogénalcényle à chaîne droite ou ramifiée ayant chacun jusqu'à 8 atomes de carbone et jusqu'à 17 atomes d'halogènes identiques ou différents, un groupe alkoxyalkyle, alkylthioalkyle, alkylsulfinyle ou alkylsulfonylalkyle à chaîne droite ou ramifiée ayant chacun 1 à 6 atomes de carbone dans les parties alkyle individuelles ou un groupe phénylalkyle ou phényle portant éventuellement dans la partie phényle 1 ou plusieurs substituants identiques ou différents, avec éventuellement 1 à 4 atomes de carbone dans la partie alkyle à chaîne droite ou ramifiée, en considérant comme substituants dans la partie phényle: un halogène, un radical cyano, nitro, un radical alkyle, alkoxy, alkylthio, alkylsulfinyle, alkylsulfonyle, halogénalkyle, halogénalkoxy, halogénalkylthio, halogénalkylsulfinyle ou halogénalkylsulfonyle à chaîne droite ou ramifiée ayant chacun 1 à 4 atomes de carbone dans les parties alkyle individuelles et, le cas échéant, 1 à 9 atomes d'halogènes identiques ou différents,

$R^3$ est un groupe alkyle ou halogénalkyle à chaîne droite ou ramifiée ayant chacun 1 à 6 atomes de carbone et, les cas échéant, 1 à 12 atomes d'halogènes identiques ou différents, un groupe cycloalkyle ayant 3 à 7 atomes de carbone ou un groupe phényle portant éventuellement un ou plusieurs substituants identiques ou différents, en considérant comme substituants du groupe phényle ceux qui sont mentionnés pour $R^2$,

Ar est un groupe phényle portant un ou plusieurs substituants identiques ou différents à l'exception du reste 4-nitrophényle ainsi que du reste 2,4-dinitrophényle,

en considérant comme substituants dans chaque cas: un radical cyano, nitro, un halogène, un radical alkyle, alkoxy ou alkoxycarbonyle à chaîne droite ou ramifiée ayant chacun 1 à 4 atomes de carbone, en outre un radical halogénalkyle ou halogénalkoxy à chaîne droite ou ramifiée ayant chacun 1 à 4 atomes de carbone et 1 à 9 atomes d'halogènes identiques ou différents ou un reste de formule $—S(O)_p—R^5$, dans laquelle

$R^5$ est un radical amino ainsi qu'un radical alkyle, alkylamino, dialkylamino ou halogénalkyle à chaîne droite ou ramifiée ayant chacun 1 à 4 atomes de carbone dans les parties alkyle individuelles et, dans le cas du radical halogénalkyle, 1 à 9 atomes d'halogènes identiques ou différents,

p est le nombre 0, 1 ou 2 et

n est le nombre 0, 1 ou 2.

2. 1-arylpyrazoles de formule (I) suivant la revendication 1, caractérisés en ce que

$R^1$ représente l'hydrogène, un groupe méthyle, éthyle, n-propyle, isopropyle ou trifluorométhyle,

$R^2$ est un groupe méthyle, éthyle, n-propyle, isopropyle, n-butyle, isobutyle, sec.-butyle, tertiobutyle, n-pentyle, isopentyle, n-hexyle, isohexyle, allyle, n-buténýle, isobutényle, n-penténýle, isopenténýle, propargyle, n-butynyle, isobutynyle, cyclopropyle, cyclobutyle, cyclopentyle, cyclohexyle, chlorométhyle, difluorométhyle, difluorochlorométhyle, fluorodichlorométhyle, trifluorométhyle, pentafluoréthyle, pentachloréthyle, fluorotétrachloréthyle, difluorotrichloréthyle, trifluorodichloréthyle, tétrafluorochloréthyle, heptafluoropropyle, chloréthyle, brométhyle, chloropropyle, bromopropyle, dichlorométhyle, chlorofluorométhyle, trichlorométhyle, dichlorométhyle, trifluoréthyle, trifluorochloréthyle, tétrafluoréthyle, difluorochloréthyle, fluorodibromométhyle, difluorobromométhyle, fluorochlorobromométhyle, chloralkyle, fluorallyle, chlorobutényle, fluorobutényle, dichlorallyle, fluorochlorallyle, difluorallyle, bromallyle, un groupe méthoxyméthyle, méthoxyéthyle, méthoxypropyle, éthoxyméthyle, éthoxyéthyle, méthylthiométhyle, méthylsulfinylméthyle, méthylsulfonyléthyle, méthylthioéthyle, méthylthiopropyle, méthylsulfinyléthyle, méthylsulfonylméthyle ou un groupe phényle, benzyle ou phényléthyle portant chacun 1 à 3 substituants identiques ou différents, en considérant comme substituants du groupe phényle: le fluor, le chlore, le brome, l'iode, un radical cyano, nitro, méthyle, éthyle, méthoxy, méthylthio, trifluorométhyle,

méthylsulfinyle, méthylsulfonyle, trifluorométhoxy, trifluorométhylthio, trifluorométhylsulfinyle ou trifluorométhylsulfonyle,

$R^3$ est un groupe méthyle, éthyle, n-propyle, isopropyle, n-butyle, isobutyle, sec.-butyle, tertiobutyle, cyclopropyl, cyclobutyle, cyclopentyle, cyclohexyle, un groupe chlorométhyle, dichlorométhyle, trichlorométhyle, bromométhyle, dibromométhyle, fluorométhyle, difluorométhyle, trifluorométhyle, fluorodichlorométhyle, difluorochlorométhyle ou un groupe phényle portant éventuellement un à trois substituants identiques ou différents, en considérant comme substituants du groupe phényle les substituants mentionnés pour $R^2$,

Ar est un reste phényle portant un à cinq substituants identiques ou différents à l'exception du reste 4-nitrophényle ainsi que du reste 2,4-dinitrophényle, en considérant comme substituants dans chaque cas: un radical cyano, nitro, fluoro, chloro, bromo, iodo, méthyle, éthyle, n-propyle, isopropyle, n-butyle, isobutyle, sec.-butyle et tertiobutyle, méthoxy, éthoxy, méthoxycarbonyle, éthoxycarbonyle, trifluorométhyle, trichlorométhyle, dichlorofluorométhyle, difluorochlorométhyle, chlorométhyle, dichlorométhyle, difluorométhyle, pentafluoréthyle, tétrafluoréthyle, trifluorochloréthyle, trifluoréthyle, difluorodichloréthyle, trifluorodichloréthyle, pentachloréthyle, trifluorométhoxy, trichlorométhoxy, dichlorofluorométhoxy, difluorochlorométhoxy, chlorométhoxy, dichlorométhoxy, difluorométhoxy, pentafluoréthoxy, tétrafluoréthoxy, trifluorochloréthoxy, trifluoréthoxy, difluordichloréthoxy, trifluorodichloréthoxy, pentachloréthoxy ou un reste de formule $—S(O)_p—R^5$, dans laquelle

$R^5$ est un radical amino, méthylamino, éthylamino, diméthylamino, diéthylamino, fluorodichlorométhyle, difluorochlorométhyle, tétrafluoréthyle, trifluorochloréthyle, trifluorométhyle, méthyle ou éthyle,

p est le nombre 0, 1 ou 2 et

n est le nombre 0, 1 ou 2.

3. Procédé de production de 1-arylpyrazoles de formule générale (I)

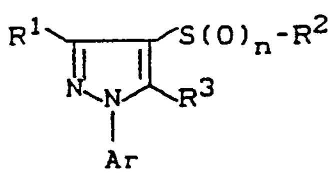

(I)

dans laquelle

$R^1$ représente l'hydrogène ou un groupe alkyle ou halogénalkyle à chaîne droite ou ramifiée ayant 1 à 4 atomes de carbone et le cas échéant 1 à 9 atomes d'halogènes identiques ou différents,

$R^2$ est un groupe alkyle, alcényle ou alcynyle à chaîne droite ou ramifiée ayant chacun jusqu'à 8 atomes de carbone, un groupe cycloalkyle de 3 à 7 atomes de carbone, un groupe halogénalkyle ou halogénalcényle, à chaîne droite ou ramifiée ayant chacun jusqu'à 8 atomes de carbone et jusqu'à 17 atomes d'halogènes identiques ou différents, un groupe alkoxyalkyle, alkylthioalkyle, alkylsulfinylalkyle ou alkylsulfonylalkyle à chaîne droite ou ramifiée ayant chacun 1 à 6 atomes de carbone dans les parties alkyle individuelles ou un groupe phénylalkyle ou phényle portant éventuellement dans la partie phényle un ou plusieurs substituants identiques ou différents, avec éventuellement 1 à 4 atomes de carbone dans la partie alkyle à chaîne droite ou ramifiée, en considérant comme substituants dans la partie phényle: un halogène, un radical cyano, nitro, un radical alkyle, alkoxy, alkylthio, alkylsulfinyle, alkylsulfonyle, halogénalkyle, halogénalkoxy, halogénalkylthio, halogénalkylsulfinyle ou halogénalkylsulfonyle à chaîne droite ou ramifiée ayant chacun 1 à 4 atomes de carbone dans les parties alkyle individuelles et, le cas échéant, 1 à 9 atomes d'halogènes identiques ou différents,

$R^3$ est un groupe alkyle ou halogénalkyle à chaîne droite ou ramifiée ayant chacun 1 à 6 atomes de carbone et, les cas échéant, 1 à 12 atomes d'halogènes identiques ou différents, un groupe cycloalkyle ayant 3 à 7 atomes de carbone ou un groupe phényle portant éventuellement un ou plusieurs substituants identiques ou différents, en considérant comme substituants du groupe phényle ceux qui sont mentionnés pour $R^2$,

Ar est un groupe phényle portant un ou plusieurs substituants identiques ou différents à l'exception du reste 4-nitrophényle ainsi que du reste 2,4-dinitrophényle, en considérant comme substituants dans chaque cas: un radical cyano, nitro, un halogène, un radical alkyle, alkoxy ou alkoxycarbonyle à chaîne droite ou ramifiée ayant chacun 1 à 4 atomes de carbone, en outre un radical halogénalkyle ou halogénalkoxy à chaîne droite ou ramifiée ayant chacun 1 à 4 atomes de carbone et 1 à 9 atomes d'halogènes identiques ou différents ou un reste de formule $—S(O)_p—R^5$, dans laquelle

$R^5$ est un radical amino ainsi qu'un radical alkyle, alkylamino, dialkylamino ou halogénalkyle à chaîne droite ou ramifiée ayant chacun 1 à 4 atomes de carbone dans les parties alkyle individuelles et, dans le cas du radical halogénalkyle, 1 à 9 atomes d'halogènes identiques ou différents,

p est le nombre 0, 1 ou 2 et

n est le nombre 0, 1 ou 2,

caractérisé en ce que, pour l'obtentioon des 1-arylpyrazoles de formule (Ia)

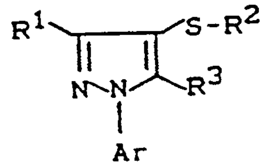

(Ia)

dans laquelle

$R^1$, $R^2$, $R^3$ et Ar ont la définition indiquée ci-dessus,

a) on fait réagir des arylhydrazines de formule (II)

$$Ar—NH—NH_2 \qquad (II)$$

dans laquelle

Ar a la définition indiquée ci-dessus, ou leurs sels d'acides avec des 1,3-dicétones de formule (IIIa)

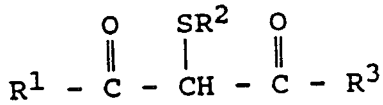

(IIIa)

dans laquelle

$R^1$, $R^2$ et $R^3$ ont la définition indiquée ci-dessus, ou bien en variante avec des cétones à non-saturation αβ de formule (IIIb)

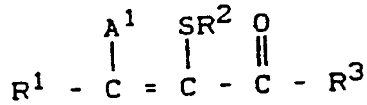

(IIIb)

dans laquelle

$R^1$, $R^2$ et $R^3$ ont la définition indiquée ci-dessus et

$A^1$ est un groupe partant tel que, par exemple, un groupe alkoxy ou dialkylamino,

le cas échéant en présence d'un diluant et en la présence éventuelle d'un catalyseur, ou bien pour obtenir les 1-arylpyrazoles de formule (Ib)

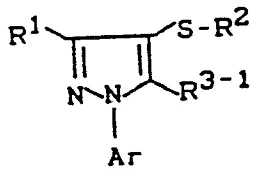

(Ib)

dans laquelle

$R^1$, $R^2$ et Ar ont la définition indiquée ci-dessus et

$R^{3-1}$ représente un groupe aryle éventuellement substitué,

b) on fait réagir des 5-amino-1-arylpyrazoles de formule (IV)

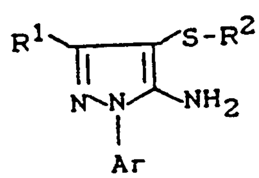

(IV)

avec des composés aromatiques de formule (V)

$$R^{3-1}—H \qquad (V)$$

dans laquelle

$R^{3-1}$ a la définition indiquée ci-dessus, en présence d'un nitrite d'alkyle de formule (VI)

$$R^4—O—N=O \qquad (VI)$$

dans laquelle

$R^4$ est un groupe alkyle

et le cas échéant en présence d'un diluant, ou bien pour obtenir les 4-sulfinyl- et 4-sulfonyl-1-arylpyrazoles de formule (Ic)

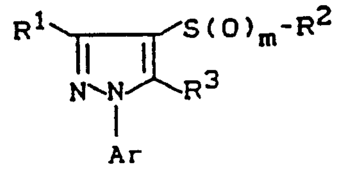

(Ic)

dans laquelle

$R^1$, $R^2$, $R^3$ et Ar ont la définition indiquée ci-dessus et

m est le nombre 1 ou 2,

c) on oxyde les 1-arylpyrazoles obtenus par le procédé (a) ou (b) de formule (Ia)

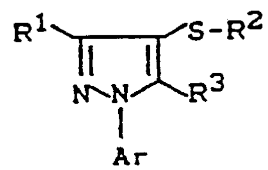

(Ia)

dans laquelle

$R^1$, $R^2$, $R^3$ et Ar ont la définition indiquée ci-dessus, avec des agents oxydants, le cas échéant en présence d'un diluant et en la présence éventuelle d'un catalyseur.

4. Compositions pesticides, caractérisées par un teneur en au moins un 1-arylpyrazole de formule (I).

5. Compositions insecticides et nématicides, caractérisées par une teneur en au moins un 1-arylpyrazole de formule (I).

6. Procédé pour combattre des insectes et/ou des nématodes, caractérisé en ce qu'on fait agir des 1-arylpyrazoles de formule (I) sur des insectes et/ou des nématodes et/ou sur leur milieu.

7. Utilisation de 1-arylpyrazoles de formule (I) pour combattre des insectes et/ou des nématodes.

8. Procédé de préparation de compositions pesticides, caractérisé en ce qu'on mélange des 1-arylpyrazoles de formule (I) avec des diluants et/ou des agents tensio-actifs.

**Claims**

1. 1-Aryl-pyrazoles of the general formula (I)

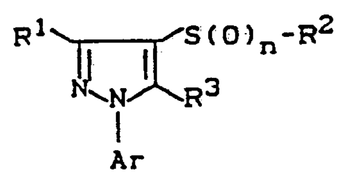

(I)

in which

$R^1$ represents hydrogen, or in each case straight-chained or branched alkyl or haloalkyl having 1 to 4 carbon atoms and optionally 1 to 9 identical or different halogen atoms,

$R^2$ represents in each case straight-chain or branched alkyl, alkenyl, or alkinyl having up to 8 carbon atoms in each case, cycloalkyl having 3 to 7 carbon atoms, in each case straight-chain or branched haloalkyl or haloalkenyl having up to 8 carbon atoms in each case and up to 17 identical or different halogen atoms, in each case straight-chain or branched alkoxyalkyl, alkylthioalkyl, alkylsulphinylalkyl or alkylsulphonylalkyl having 1 to 6 carbon atoms in each of the individual alkyl moieties, or phenylalkyl or phenyl, optionally having 1 to 4 carbon atoms in the straight-chain or branched alkyl moiety, which is in each case optionally mono- or poly-substituted in the phenyl moiety, the substituents being identical or different and suitable substituents in the phenyl moiety being: halogen, cyano, nitro, in each case straight-chain or branched alkyl, alkoxy, alkylthio, alkylsulphinyl, alkylsulphonyl, haloalkyl, haloalkoxy, haloalkylthio, haloalkylsulphinyl or haloalkylsulphonyl having 1 to 4 carbon atoms in each of the individual alkyl moieties and optionally having 1 to 9 identical or different halogen atoms,

$R^3$ represents in each case straight-chain or branched alkyl or haloalkyl having 1 to 6 carbon atoms in each case and optionally having 1 to 12 identical or different halogen atoms, cycloalkyl having 3 to 7 carbon atoms or optionally mono- or polysubstituted phenyl, the substituents being identical or different and suitable phenyl substituents being those mentioned for $R^2$,

Ar represents mono- or polysubstituted phenyl, with the exception of the 4-nitrophenyl or the 2,4-dinitrophenyl radical, the substituents being identical or different and suitable substituents in each case being: cyano, nitro, halogen, in each case straight-chain or branched alkyl, alkoxy or alkoxy-carbonyl having 1 to 4 carbon atoms in each case, in addition in each case straight-chain or branched haloalkyl or haloalkoxy having 1 to 4 carbon atoms in each case and 1 to 9 identical or different halogen atoms, or a $-S(O)_p-R^5$ radical, where

$R^5$ represents amino and also in each case straight-chain or branched alkyl, alkylamino, dialkylamino or haloalkyl having 1 to 4 carbon atoms in each of the individual alkyl moieties and, in the case of haloalkyl, having 1 to 9 identical or different halogen atoms,

p represents a number 0, 1 or 2 and

n represents a number 0, 1 or 2.

2. 1-Aryl-pyrazoles of the formula (I) according to Claim 1, characterized in that

$R^1$ represents hydrogen, methyl, ethyl, n- or i-propyl or trifluoromethyl,

$R^2$ represents methyl, ethyl, n- or i-propyl, n-, i-, s- or t-butyl, n- or i-pentyl, n- or i-hexyl, allyl, n- or i-butenyl, n- or i-pentenyl, propargyl, n- or i-butinyl, cyclopropyl, cyclobutyl, cyclopentyl, cyclohexyl, chloromethyl, difluoromethyl, difluorochloromethyl, fluorodichloromethyl, trifluoromethyl, pentafluoroethyl, pentachloroethyl, fluorotetrachloroethyl, difluorotrichloroethyl, trifluorodichloroethyl, tetrafluorochloroethyl, heptafluoropropyl, chloroethyl, bromoethyl, chloropropyl, bromopropyl, dichloromethyl, chlorofluoromethyl, trichloromethyl, dichloromethyl, trifluoroethyl, trifluorochloroethyl, tetrafluoroethyl, difluorochloroethyl, fluorodibromomethyl, difluorobromomethyl, fluorochlorobromomethyl, chloroalkyl, fluoroallyl, chlorobutenyl, fluorobutenyl, dichloroallyl, fluorochloroallyl, difluoroallyl, bromoallyl, methoxymethyl, methoxyethyl, methoxypropyl, ethoxymethyl, ethoxyethyl, methylthiomethyl, methylsulphinylmethyl, methylsulphonylethyl, methylthioethyl, methylthiopropyl, methylsulphinylethyl, methylsulphonylmethyl or in each case optionally mono- to trisubstituted phenyl, benzyl or phenylethyl, the substituents being identical or different and suitable phenyl substituents being: fluorine, chlorine, bromine, iodine, cyano, nitro, methyl, ethyl, methoxy, methylthio, trifluoromethyl, methylsulphinyl, methylsulphonyl, trifluoromethoxy, trifluoromethylthio, trifluoromethylsulphinyl or trifluoromethylsulphonyl,

$R^3$ represents methyl, ethyl, n- or i-propyl, n-, i-, s- or t-butyl, cyclopropyl, cyclobutyl, cyclopentyl, cyclohexyl, chloromethyl, dichloromethyl, trichloromethyl, bromomethyl, dibromomethyl, fluoromethyl, difluoromethyl, trifluoromethyl, fluorodichloromethyl, difluorochloromethyl or optionally mono- to trisubstituted phenyl, the substituents being identical or different and suitable phenyl substituents being those mentioned for $R^2$,

Ar represents mono- to pentasubstituted phenyl, with the exception of the 4-nitrophenyl radical or the 2-4-dinitrophenyl radical, the substituents being identical or different and suitable substituents in each case being: cyano, nitro, fluorine, chlorine, bromine, iodine, methyl, ethyl, n- or i-propyl, n-, i-, s- and t-butyl, methoxy, ethoxy, methoxycarbonyl, ethoxycarbonyl, trifluoromethyl, trichloromethyl, dichlorofluoromethyl, difluorochloromethyl, chloromethyl, dichloromethyl, difluoromethyl, pentafluoroethyl, tetrafluoroethyl, trifluorochloroethyl, trifluoroethyl, difluorodichloroethyl, trifluorodichloroethyl, pentachloroethyl, trifluoromethoxy, trichloromethoxy, dichlorofluoromethoxy, difluorochloromethoxy, chloromethoxy, dichloromethoxy, difluoromethoxy, pentafluoroethoxy, tetrafluoroethoxy, trifluorochloroethoxy, trifluoroethoxy, difluorodichloroethoxy, trifluorodichloroethoxy, pentachloroethoxy or a $-S(O)_p-R^5$ radical, where

$R^5$ represents amino, methylamino, ethylamino, dimethylamino, diethylamino, fluorodichloromethyl, difluorochloromethyl, tetrafluoroethyl, trifluorochloroethyl, trifluoromethyl, methyl or ethyl,

p represents a number 0, 1 or 2 and

n represents a number 0, 1 or 2.

3. Process for the preparation of 1-aryl-pyrazoles of the general formula (I)

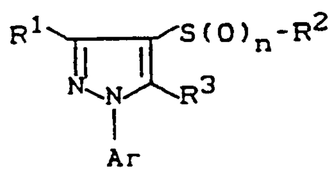

(I)

in which

$R^1$ represents hydrogen, or in each case straight-chain or branched alkyl or haloalkyl having 1 to 4 carbon atoms and optionally 1 to 9 identical or different halogen atoms,

$R^2$ represents in each case straight-chain or branched alkyl, alkenyl, or alkinyl having up to 8 carbon atoms in each case, cycloalkyl having 3 to 7 carbon atoms, in each case straight-chain or branched haloalkyl or haloalkenyl having up to 8 carbon atoms in each case and up to 17 identical or different halogen atoms, in each case straight-chain or branched alkoxyalkyl, alkylthioalkyl, alkylsulphinylalkyl or alkylsulphonylalkyl having 1 to 6 carbon atoms in each of the individual alkyl moieties, or phenylalkyl or phenyl, optionally having 1 to 4 carbon atoms in the straight-chain or branched alkyl moiety, which is in each case optionally mono- or polysubstituted in the phenyl moiety, the substituents being identical or different and suitable

substituents in the phenyl moiety being: halogen, cyano, nitro, in each case straight-chain or branched alkyl, alkoxy, alkylthio, alkylsulphinyl, alkylsulphonyl, haloalkyl, haloalkoxy, haloalkylthio, haloalkylsulphinyl or haloalkylsulphonyl having 1 to 4 carbon atoms in each of the individual alkyl moieties and optionally having 1 to 9 identical or different halogen atoms,

$R^3$ represents in each case straight-chain or branched alkyl or haloalkyl having 1 to 6 carbon atoms in each case and optionally having 1 to 12 identical or different halogen atoms, cycloalkyl having 3 to 7 carbon atoms or optionally mono- or polysubstituted phenyl, the substituents being identical or different and suitable phenyl substituents being those mentioned for $R^2$,

Ar represents mono- or polysubstituted phenyl, with the exception of the 4-nitrophenyl or the 2,4-dinitrophenyl radical, the substituents being identical or different and suitable substituents in each case being: cyano, nitro, halogen, in each case straight-chain or branched alkyl, alkoxy or alkoxy-carbonyl having 1 to 4 carbon atoms in each case, in addition in each case straight-chain or branched haloalkyl or haloalkoxy having 1 to 4 carbon atoms in each case and 1 to 9 identical or different halogen atoms, or a $—S(O)_p—R^5$ radical, where

$R^5$ represents amino and also in each case straight-chain or branched alkyl, alkylamino, dialkylamino or haloalkyl having 1 to 4 carbon atoms in each of the individual alkyl moieties and, in the case of haloalkyl having 1 to 9 identical or different halogen atoms,

p represents a number 0, 1 or 2 and

n represents a number 0, 1 or 2,

characterized in that, to obtain the 1-aryl-pyrazoles of the formula (Ia),

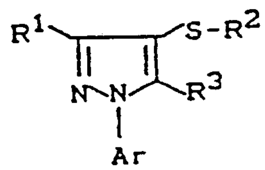

(Ia)

in which

$R^1$, $R^2$, $R^3$ and Ar have the abovementioned meaning,

a) arylhydrazines of the formula (II)

$$Ar—NH—NH_2 \quad (II)$$

in which

Ar has the abovementioned meaning, or their hydrates, are reacted with 1,3-diketones of the formula (IIIa)

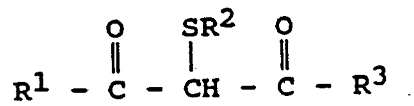

(IIIa)

in which

$R^1$, $R^2$ and $R^3$ have the abovementioned meaning, or, alternatively, are reacted with αβ-unsaturated ketones of the formula (IIIb),

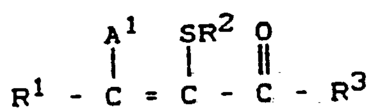

(IIIb)

in which

$R^1$, $R^2$ and $R^3$ have the abovementioned meaning and

$A^1$ represents a leaving group, such as, for example, alkoxy or dialkylamino,

if appropriate in the presence of a diluent and if appropriate in the presence of a catalyst, or in that, to obtain the 1-arylpyrazoles of the formula (Ib),

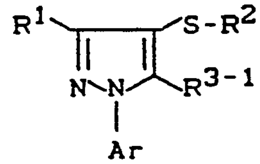

(Ib)

in which

$R^1$, $R^2$ and Ar have the abovementioned meaning and

$R^{3-1}$ represents optionally substituted aryl,

b) 5-amino-1-aryl-pyrazoles of the formula (IV),

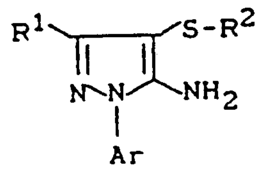

(IV)

are reacted with aromatics of the formula (V),

$$R^{3-1}—H \qquad (V)$$

in which

$R^{3-1}$ has the abovementioned meaning, in the presence of an alkyl nitrite of the formula (VI),

$$R^4—O—N=O \qquad (VI)$$

in which

$R^4$ represents alkyl

and if appropriate in the presence of a diluent, or in that, to obtain the 4-sulphinyl- and 4-sulphonyl-1-arylpyrazoles of the formula (Ic),

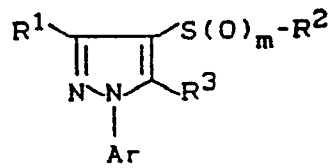

(Ic)

in which

$R^1$, $R^2$, $R^3$ and Ar have the abovementioned meaning and

m represents a number 1 or 2,

c) the 1-arylpyrazoles of the formula (Ia),

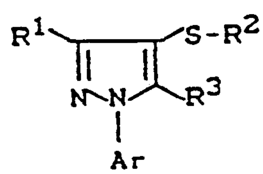

(Ia)

in which

$R^1$, $R^2$, $R^3$ and Ar have the abovementioned meaning, which are obtained by process (a) or (b), are oxidized using oxidants, if appropriate in the presence of a diluent and if appropriate in the presence of a catalyst.

4. Pesticides, characterized in that they contain at least one 1-aryl-pyrazole of the formula (I).

5. Insecticides and nematicides, characterized in that they contain at least one 1-aryl-pyrazole of the formula (I).

6. Process for combating insects and/or nematodes, characterized in that 1-aryl-pyrazoles of the formula (I) are allowed to act on insects and/or namatodes and/or on their habitat.

7. Use of 1-aryl-pyrazoles of the formula (I) for combating insects and/or nematodes.

8. Process for the preparation of pesticides, characterized in that 1-aryl-pyrazoles of the formula (I) are mixed with extenders and/or surfactants.